# FASCICULE DE BREVET EUROPEEN

(11) **EP 1 268 397 B1**
(45) Date de publication et mention de la délivrance du brevet: **05.12.2007**
(21) Numéro de dépôt: 01919554.4
(22) Date de dépôt: 23.03.2001
(51) Int. Cl.: C07C 227/32, C07C 229/22, C07C 255/26, C07D 307/32, C07C 313/06, C07C 271/22

(54) **PROCEDE DE PREPARATION DE LA (2S,3R,4S)-4-HYDROXYISOLEUCINE ET DE SES ANALOGUES**
VERFAHREN ZUR HERSTELLUNG VON (2S,3R,4S)-4-HYDROXYISOLEUCIN UND ANALOGA
METHOD FOR PREPARING (2S,3R,4S)-4-HYDROXYISOLEUCINE AND ANALOGUES THEREOF

(30) Priorité: 27.03.2000 FR 0003859
(43) Date de publication de la demande: 02.01.2003
(73) Titulaire: CENTRE NATIONAL DE LA RECHERCHE SCIENTIFIQUE (CNRS), 75794 Paris Cedex 16 (FR)
(72) Inventeur: OUAZZANI, Jamal, F-91300 Massy (FR); POTIER, Pierre, F-75007 Paris (FR); SASAKI, Nobumichi, André, F-91940 les Ulis (FR); ZHU, Wang, Qian, F-91190 Gif-sur-Yvette (FR)
(74) Mandataire: Ahner, Francis
(86) Numéro de dépôt international: PCT/FR2001/000885
(87) Numéro de publication internationale: WO 2001/072688

(56) Documents cités:
- WO-A-97/32577

## Description

La présente invention concerne des procédés de préparation d'acides aminés α.

Elle concerne en particulier un procédé de préparation d'acide aminé α de Formule générale (2S)-1 suivante : 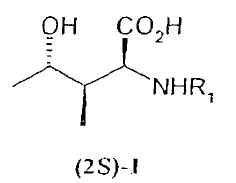 dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle.

Elle concerne également les acides aminés α de formule générale (25)-I dans laquelle le groupe R₁ représente un groupe de formule -COOR₂ dans laquelle le groupe R₂ représente un groupe alkyle en C₁-C₆, un groupe aryle ou aralkyle.

En 1973, Fowden *et al.* ont rapporte la présence de l'acide (2S,3R,4R)-4-hydroxy-3-méthylpentanoïque (4-hydroxyisoleucine) 1 dans le fenugrec (*Trigonella foenum-graecum)* qui est une plante herbacée annuelle largement répandue dans des régions d'Asie, d'Afrique et d'Europe (Fowden et al. Phytochemistry 1973, 12, 1707-1711). Sa configuration absolue a été réétudiée ultérieurement et corrigée comme étant (2S,3R,4S) par Alcock *et al.* en 1989 *(*Phytochemistry 1989, 28, 1835-1841). Cet acide aminé α de formule suivante : 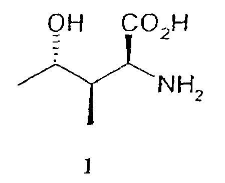 s'est également avéré être un composant de la γ-amanitine, un peptide cyclique mortellement toxique produit dans l*'Amanita phalloide* (Wieland et al. Liebigs Ann. Chem. 1970, 736, 95-99; Gieren et al. Liebigs Ann. Chem. 1974, 1561-1569 et Hasan et al. Liebigs Ann. Chem. 1976, 781-787), Il a été démontré que la (2S,3R,4S)-4-hydroxyisoleucine **1** possède une activité insulinotrope (Travis et al. dans "The Pharmacological Basis of Therapeutics", Goodman L.S. et Gilman A. éd., 1970, p 1581. MacMillan, Londres: Sauvaire et al. Diabetes 1998, 47, 206-210; Petit et al. Diabetologia 1995, 38, A101; Fernandez-Alvarez et al. Diabetologia 1996, 39, A234; Ribes et al. Diabetologia 1996, 39. A234; Petit et al. Diabetologia 1997, 40, A112; Broca et al. Diabetologia, 1998, 41, A239; Broca et al. Diabetologia 1999, 42, A 129 et la demande internationale PCT WO9732577). Malgré cette découverte que **1** en tant que nouveau médicament avait un intérêt potentiel pour le traitement du diabète méllitusé non insulino-dependant, on n'a divulgué jusqu'ici que la synthèse asymétrique des diastéréoméres (2R,3R,4R) et (2S,3R,4R) (Inghardt et al. Tetrahedron 1991, 47, 6469-6482). 1 en revanche n'est obtenu que par l'extraction à partir des graines de fenugrec qui est un processus très laborieux et coûteux (plantation, entretien, recette, transport, extraction, etc.). En particulier, l'élimination de la glycine, l'acide aminé concomitant, est extrêmement difficile dans le processus d'extraction (Sauvaire et al. Phytochemistry 1984, 23, 479-486) On rapporte que le rendement global en 1 à partir de matières végétales séchées est de 0,56% p/p (Sauvaire et al. Diabetes 1998, 47, 206-210) L'article de Blank et al (J. Agric. Food. Chem. 1996, 44, 1851-1856) décrit la préparation de la (2S, 3R, 4S) 4-hydroxyisoleucine par hydrolyse alcaline de la (2S, 3R, 4S)-aminolactone correspondante. Toutefois, l'hydrolyse diastérolictive des lactones de formules générales VI et VII et l'hydrolyse alcaline de la lactone de formule IX n'y sont pas décrites. EN conséquence il y a toujours nécessité d'avoir un procédé facile et économique pour obtenir **1**. Or, de façon surprenante la méthode de synthèse asymétrique décrite dans la présente invention permet de fournir la (2S,3R,4S)-4-hydroxyisoleucine 1 et ses analogues de formule **(2S)-I** en quantité industrielle et de qualité optiquement pure de façon économiquement très intéressante et très pratique.

Les acides aminés a de formule générale **(2S)-I** suivante : 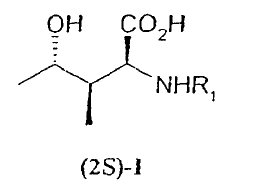 dans laquelle le groupe R₁ représente un groupe de formule -COOR₂ dans laquelle le groupe R₂ représente un groupe alkyle en C₁-C₆, un groupe aryle ou aralkyle n'ayant jamais été synthétisés sont donc nouveaux et font également partis de l'invention,

La présente invention concerne donc un procédé de synthèse de composés de formule générale **(2S)-I** dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle.

Par le terme « groupe protecteur du groupe amine », on entend au sens de la présente invention tout groupe alkyle en C₁-C₆, tout groupe aryle ou aralkyle et surtout tout groupe labile protégeant la fonction amine et bien connu de l'homme du métier, en particulier du type, benzyle, (S)-(+)-p-toluènesulfino 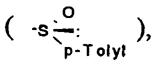 (S)-1-phényléthyle (-CH(CH₃)Ph) et (S)-1-phényl-2-hydroxyéthyle (-CH(CH₂OH)Ph).

Par le terme « groupe alkyle en C₁-C₆ », on entend au sens de la présente invention tout groupe alkyle en C₁-C₆ linéaire ou ramifié, substitué ou non, en particulier, les groupes méthyle, éthyle ou t-butyle.

Par le terme « groupe aryle », on entend au sens de la présente invention un ou plusieurs cycles aromatiques ayant 5 à 8 atomes de carbones, pouvant être accolés ou fusionnés, substitués ou non. En particulier, les groupes aryles peuvent être des groupes phényles ou naphthyles.

Par le terme « groupe aralkyle », on entend au sens de la présente invention des groupes aryles tels que définis ci-dessus, liés par l'intermédiaire d'un groupe alkyle tel que défini ci-dessus. En particulier un groupe aralkyle est un groupe benzyle.

Ce procédé comporte en particulier les étapes de:
-a) réduction diastéréo et énantiosélective du 2-méthylacétoacétate d'éthyle de formule 2 suivante : 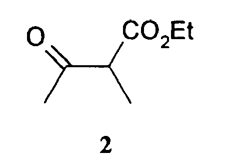 pour obtenir le composé de formule 3 suivante : 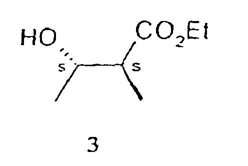 avec un excès énantiomérique (ee) d'au moins 85%, avantageusement d'au moins 90%, et un excès diastéréomérique (de) d'au moins 90%, avantageusement d'au moins 95%. Le de est déterminé par analyse chromatographique en phase gazeuse (CPG) et le ee est estimé par les spectres RMN ¹H et ¹³C du (S)-lactate d'éthyle de 3.
   De façon avantageuse la réduction diastéréo et énantiosélective se fait par utilisation d'une enzyme, de façon encore plus avantageuse par utilisation d'un micro-organisme contenant cette enzyme, en particulier le *Geotrichum candidum* (Buisson et al. Tetrahedron Lett. 1987, 28, 3939-3940 et Kawai et al. Tetrahedron Lett. 1994, 35, 147-148).
   L'enzyme utilisée peut être en particulier isolée à partir du *Geotrichum candidum* (déposé à la Collection Nationale de Culture de Microorganismes le 8 décembre 1999 avec le numéro d'enregistrement: I-2366) et utilisée sous forme de poudre ou fixée sur un support.
-b) protection du groupe OH de l'alcool de formule 3 par un groupe Y. Au sens de la présente invention, on entend par « groupe protecteur du groupe OH » tout groupe connu comme tel par l'homme du métier, en particulier les groupes benzyle (Bn), *t*-butyle ou tétrahydropyranne (THP).
-c) réduction en alcool du groupe -COOEt du composé obtenu en b). La réduction peut se faire par toute méthode bien connue par l'homme du métier, en particulier par utilisation de LiAIH₄ ou d'hydrure de diisobutyl aluminium (DIBAL).
-d) oxydation du groupe OH non protégé obtenu en c) pour donner l'aldéhyde de formule générale **II** suivante : 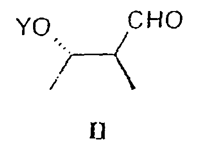
dans laquelle le groupe Y représente un groupe protecteur du groupe OH.

L'oxydation peut se faire par toute méthode bien connue de l'homme du métier et notamment par traitement avec un complexe pyridine/trioxyde de soufre (Py·SO₃) dans du diméthylesulfoxyde (DMSO), par oxydation de Swem ou par traitement avec le 2,2,6,6-tétraméthyl-1-pipéridinyloxy à radical libre (TEMPO) en utilisant NaOCl comme agent réducteur en présence de NaBr/NaHCO₃/H₂O.

A partir du composé de formule générale **II** deux voies de synthèse des composés de formule générale **(2S)-I** sont possibles :
La première voie de synthèse (méthode A) est une approche asymétrique et comporte les étapes e₁) à h₁) suivantes :
-e₁) traitement du composé de formule générale **II** avec une sulfinamide de configuration (S) (+) pour obtenir le composé de formule générale **IV** suivante : 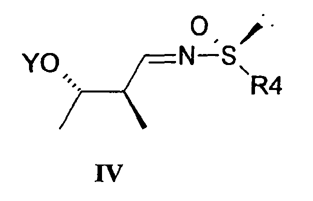 dans laquelle
   le groupe Y représente un groupe protecteur du groupe OH
   et le groupe R4 représente un groupe alkyle en C₁-C₆, aryle ou aralkyle.
   Les sulfinamides de configuration (S) (+) qui peuvent être utilisées sont en particulier celles pour lesquelles le groupe R4 représente le groupe t-butyle, 2-méthoxy-1-naphthalényle, 2-[1-(t-butylcarbonylamino)éthyl]benzyle ou p-toluène.
   La (S)-(+)-p-toluènesulfinamide peut être préparée facilement à partir du (1R,2S,5R)-(-)-menthyl-(S)-p-toluènesulfinate et du 1,1,1,3,3,3-héxaméthyle-disiloxane de lithium (LiHMDS).
-f₁) traitement du composé de formule générale IV, en particulier avec du cyanure d'éthylisopropyloxyaluminium (EtAI(OiPr)CN), pour obtenir l'aminonitrile de formule générale V suivante : 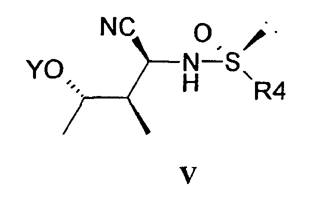 dans laquelle
   le groupe Y représente un groupe protecteur du groupe OH
   et le groupe R4 représente un groupe alkyle en C₁-C₆, aryle ou aralkyle.
g₁) traitement avec un acide, en particulier par mise à reflux dans un acide minéral de type HCl ou HBr, de l'aminonitrile de formule générale **V** pour obtenir le sel de la lactone de formule 9 suivante : 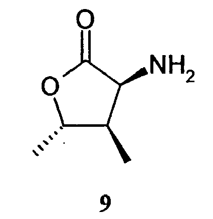 On entend par sel de lactone, les sels obtenus suite au traitement avec l'acide minéral, en particulier des chlorures ou des bromures.
   On peut également, par une autre variante du procédé, obtenir des aminonitriles à l'étape f₁) et des lactones à l'étape g₁) de bonnes configurations par une synthèse asymétrique utilisant des amines chirales pour l'induction asymétrique en position C₂ des composés de formule générale **II.** Ces procédés sont bien connus de l'homme de métier et utilisent en particulier comme amines chirales la (S)-α-méthybenzylamine, le (S)-α-phénylglycinol, le (S)-α-N-benzylphénylglycinol, la (S)-α-butylbenzylamine, la (S)-α-éthylbenzylamine, la (S)-α-naphthylbenzylamine, le (4S,5S)-(+)-5-amino-2,2-diméthyl-4-phényl-1,3-dioxane ou la 2,3,4,6-tétra-O-pivaloyl-β-D-galactopyranosylamine.
-h₁) hydrolyse alcaline, en particulier avec LiOH, du sel de la lactone de formule 9 pour obtenir le composé de formule générale **(2S)-I** dans lequel le groupe R₁ représente l'atome d'hydrogène. Ce composé peut être purifié, si nécessaire, par résine échangeuse d'ions Dowex 50WX8 (forme H+), par neutralisation avec un acide organique, en particulier l'acide formique ou l'acide acétique, et cristallisation dans un alcool, en particulier dans l'isopropanol, le propanol ou l'éthanol, ou par toute autre méthode connue de l'homme du métier.
   En variante, la deuxième voie de synthèse (méthode B) à partir du composé de formule générale **II** comporte les étapes e₂) à h₂) suivantes :
-e₂) traitement du composé de formule générale **II** pour obtenir l'aminonitrile de formule générale **III** suivante : 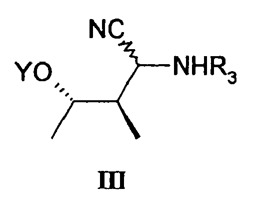 dans laquelle :
   le groupe Y représente un groupe protecteur du groupe OH
   et le groupe R₃ représente un groupe protecteur du groupe amine.
   En particulier, pour obtenir le composé de formule générale **III** dans lequel le groupe R₃ représente le groupe benzyle (Bn), on traite le composé de formule générale **II** avec de préférence du chlorhydrate de benzylamine et du KCN, de préférence en quantité équimolaire, ou de façon encore plus préférée avec la benzylamine et du cyanure de triméthylsilyle (TMSCN). L'aminonitrile obtenu est un mélange racémique des diastéréomères **(1S)** et **(1R),** en particulier avec un rapport **(1S):(1R)** de 55:45.
   De préférence, pour obtenir le composé de formule générale **III** dans lequel le groupe R₃ représente le groupe (-CH(CH₃)Ph), on peut traiter le composé de formule générale **II** avec, de préférence, le (S)-NH₂CH(CH₃)Ph et du TMSCN ou le sel de (S)-NH₂CH(CH₃)Ph et du KCN. L'aminonitrile obtenu est un mélange de diastéréomères avec en particulier un rapport **(1S):(1R)** de 3,5:1 à 4:1.
-f₂) traitement avec un acide, en particulier par mise à reflux dans un acide minéral de type HCl ou HBr, de l'aminonitrile de formule générale **III** pour obtenir la lactone de formule générale **VI** suivante : 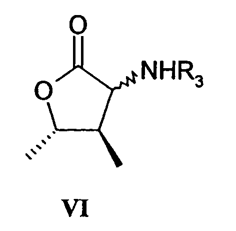 dans laquelle le groupe R₃ représente l'atome d'hydrogène ou un groupe protecteur du groupe amine. Le rendement de cette réaction est quantitatif.
   L'étape f₂) peut être suivie par une étape supplémentaire f_{2,1}) de traitement de la lactone de formule générale **VI** pour obtenir la lactone de formule générale **VII** suivante : 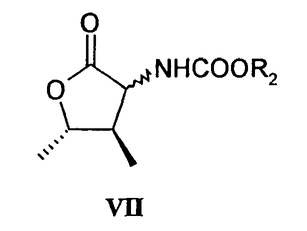 dans laquelle le groupe R₂ représente un groupe alkyle en C₁-C₆, un groupe aryle ou aralkyle.
   Ce traitement de la lactone de formule générale **VI** se fait de façon connue par l'homme du métier et notamment en utilisant de l'hydrogène en présence d'un catalyseur, avantageusement contenant du palladium et de façon encore plus avantageuse avec le catalyseur Pd-C, et un composé A comportant un groupe -COOR₂ dans lequel le groupe R₂ représente un groupe alkyle en C₁-C₆, un groupe aryle ou aralkyle. Avantageusement, dans le cas où on souhaite préparer un composé de formule **VII** dans laquelle le groupe R₂ représente un groupe méthyle, le composé A est le (COOCH₃)₂O, dans celui où le groupe R₂ représente un groupe t-butyle, le composé A est le bicarbonate de di-tert-butyle et dans celui où le groupe R₂ représente le groupe benzyle, le composé A est le chlorure de benzyloxycarbonyle.
-g₂) hydrolyse diastéréosélective de la lactone de formule générale **VI** ou **VII** pour obtenir le composé de formule générale **(2S)-I.**
   Cette étape g₂) peut en particulier consister en trois variantes.
   La première variante g_{2,1}) ne concerne que les lactones de formule générale **VII** ou les lactones de formule générale **VI** dans laquelle le groupe R₃ représente un groupe protecteur du groupe amine. Elle consiste alors dans les étapes g_{2,1,1}) à g_{2,1,3}):
-g_{2,1,1}) l'hydrolyse alcaline, en particulier avec LiOH hydraté dans l'eau, de la lactone pour obtenir un mélange de diastéréomères de formule générale **I** suivante : 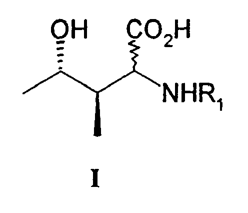 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle. Ce mélange comporte de préférence un rapport 7:3 en faveur de l'isomère **(2S).** Cette hydrolyse peut être suivie par chromatographie en couche mince (CCM) jusqu'à son achèvement et peut se faire avec agitation pendant 1 heure à température ambiante.
-g_{2,1,2}) l'ajout d'acide organique, de préférence l'acide trifluoroacétique (TFA), sous forme de traces, de préférence 2% v/v, et le chauffage, de préférence à une température comprise entre environ 40 et environ 80°C, encore plus préférentiellement entre environ 50 et environ 60°C, pour recycliser le composé de formule générale **(2R)-I** suivante : 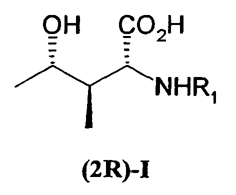 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
   en lactone de formule générale **(3R)-VIII** suivante : 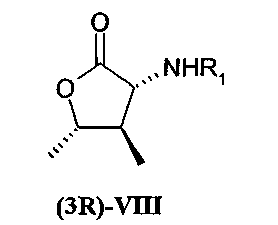 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle. La recyclisation peut être suivie par chromatographie en phase liquide à haute performance (HPLC).
-g_{2,1,3}) l'extraction avec un solvant organique, en particulier avec de l'acétate d'éthyle (EtOAc) et de façon encore plus particulière avec un mélange EtOAc/heptane dans un rapport 1:1, de la lactone de formule générale **(3R)- VITI** pour récupérer le composé de formule générale **(2S)-I** dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle.
   La deuxième variante g₂,₂) consiste dans les étapes g_{2,2,1}) à g_{2,2,3}) :
-g_{2,2,1}) l'hydrolyse enzymatique diastéréosélective de la lactone de formule générale **(3R)-VI** ou **(3R)-VII** par des hydrolases pour donner le composé de formule générale **(2R)-I.** Ces hydrolases peuvent être utilisée sous forme purifiée, partiellement purifiée, ou produits *in situ* par des microorganismes.
   En particulier, on peut utiliser des préparations enzymatiques brutes ou des cellules microbiennes vivantes. Avantageusement, on utilise pour réaliser cette hydrolyse une poudre acétonique de foie de porc, commercialisée par la société Sigma sous la référence L.8251 ou la souche microbienne *Penicillium* (déposée le 29 septembre 1998 à la Collection Nationale de Cultures de Microorganismes avec le numéro d'enregistrement : I-2081).
-g_{2,2,2}) l'extraction avec un solvant organique, de préférence EtOAc, de la lactone non hydrolysée restante de formule générale **(3S)-VIII** suivante : 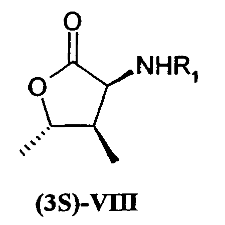 dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle,
-g_{2,2,3}) l'hydrolyse enzymatique non sélective de la lactone de formule générale **(38)-VIII** pour obtenir le composé de formule générale **(2S)-I** dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle.
   L'étape g₂) peut être suivie par une étape supplémentaire h₂) dans le cas où le groupe R₁ du composé de formule générale **(2S)-1** représente un groupe protecteur du groupe amine ou un groupe de formule -COOR₂ dans laquelle le groupe R₂ représente un groupe alkyle en C₁-C₆, un groupe aryle ou aralkyle permettant l'obtention de la (2S,3R,4S)-4-hydroxyisoleucine **1.** Cette étape supplémentaire consiste en une hydrogénolyse catalytique, en particulier par utilisation d'un catalyseur à base de palladium, de façon encore plus particulière par l'utilisation du catalyseur Pd/C, du composé de formule **(2S)-I** dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle.
   La troisième variante g_{2,3}) ne concerne que les lactones de formule générale VI dans laquelle le groupe R₃ représente un groupe protecteur du groupe amine, en particulier un groupe (-CH(CH₃)Ph). Elle consiste alors dans les étapes g_{2,3,1}) à g_{2,3,3}):
-g₂,₃,_{I}) cristallisation dans un solvant organique, avantageusement de l'isopropanol, de la lactone de formule générale VI dans laquelle le groupe R₃ représente un groupe protecteur du groupe amine de façon à obtenir le sel du composé de formule (3S)-VIII dans laquelle le groupe R₁ représente un groupe protecteur du groupe amine,
-g_{2,3,2}) hydrogénolyse catalytique, en particulier par utilisation d'un catalyseur à base de palladium, de façon encore plus particulière par l'utilisation du catalyseur Pd/C, du sel du composé de formule (3S)-VIII dans laquelle le groupe R₁ représente un groupe protecteur du groupe amine de façon à obtenir le sel de la lactone de formule 9,
-g_{2,3,3}) hydrolyse alcaline, en particulier avec LiOH, du sel de la lactone de formule 9 pour obtenir le composé de formule générale (2S)-I dans lequel le groupe R₁ représente l'atome d'hydrogène. Ce composé peut être purifié, si nécessaire, par résine échangeuse d'ions, en particulier Dowex 50WX8 (forme H+), par neutralisation avec un acide organique, en particulier l'acide formique ou l'acide acétique, et cristallisation dans un alcool, en particulier dans l'isopropanol, le propanol ou l'éthanol, ou par toute autre méthode connue de l'homme du métier.

Le procédé selon l'invention peut être illustré à titre indicatif et de façon non limitative par le schéma réactionnel de synthèse de la (2S,3R,4S)-4-hydroxyisoleucine **1** suivant :

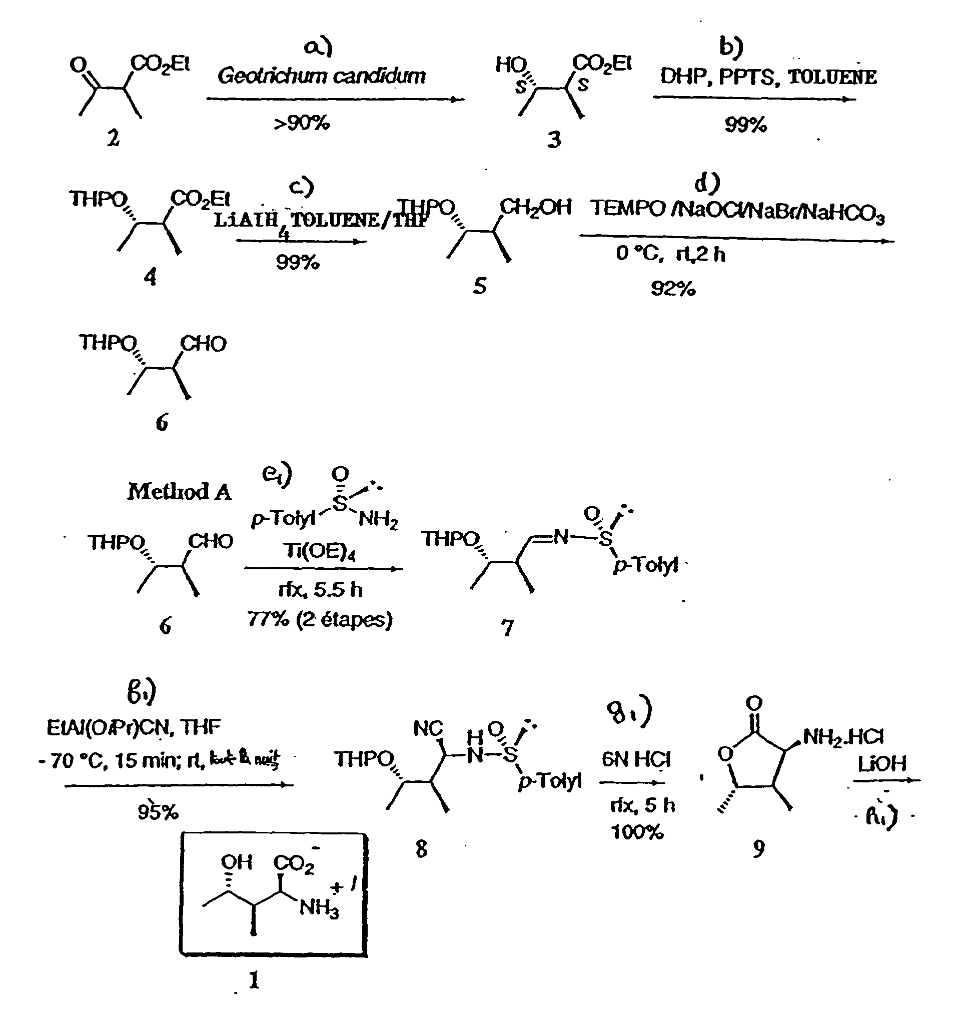

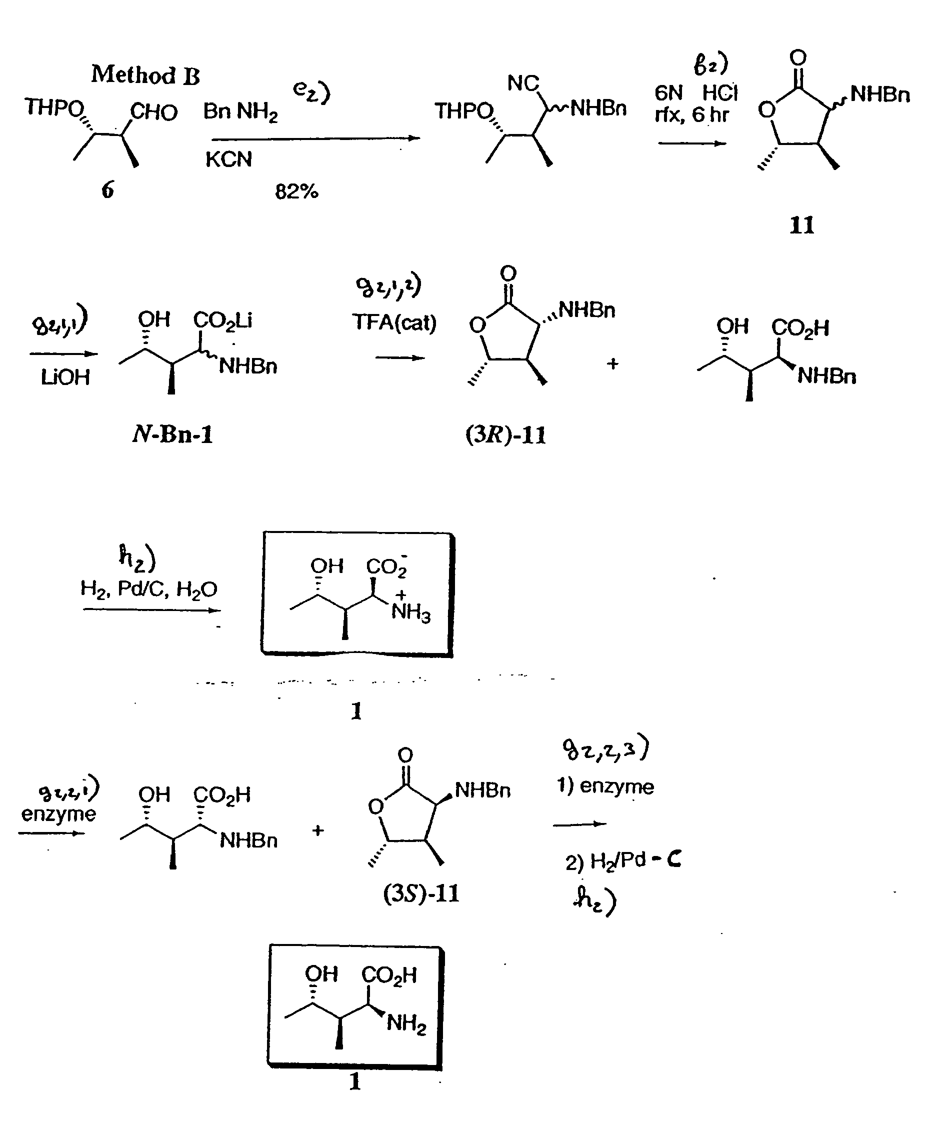

En variante, la méthode B de synthèse de la (2S,3R,4S)-4-hydroxyisoleucine **1** peut avoir la schéma réactionnel suivant :

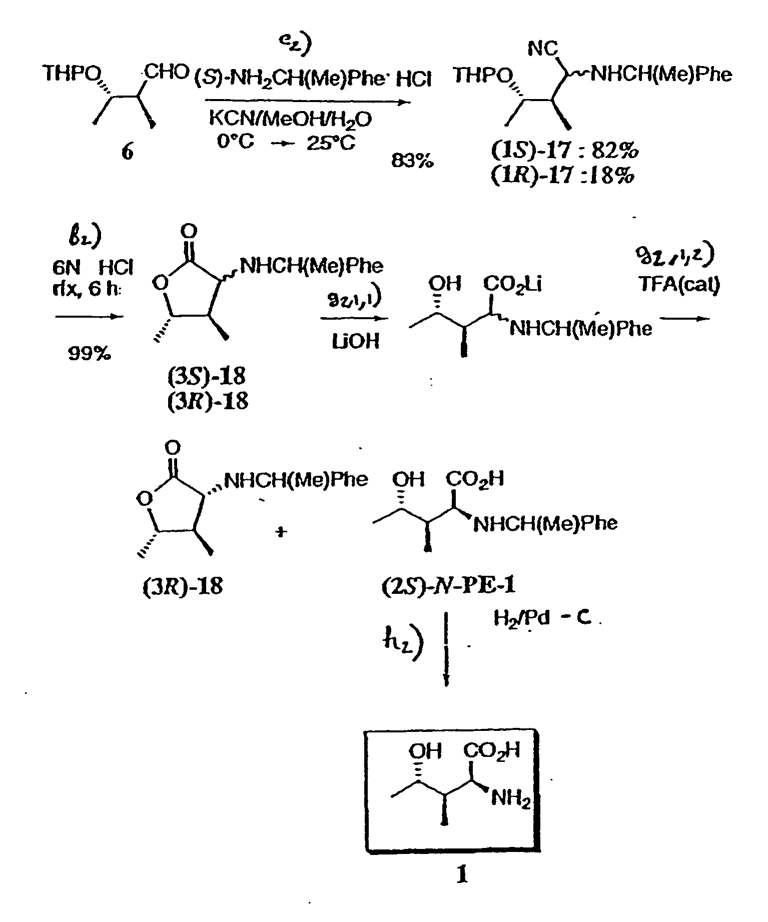

En variante, la méthode B de synthèse de la (2S,3R,4S)-4-hydroxyisoleueine 1 peut également avoir la schéma réactionnel suivant :

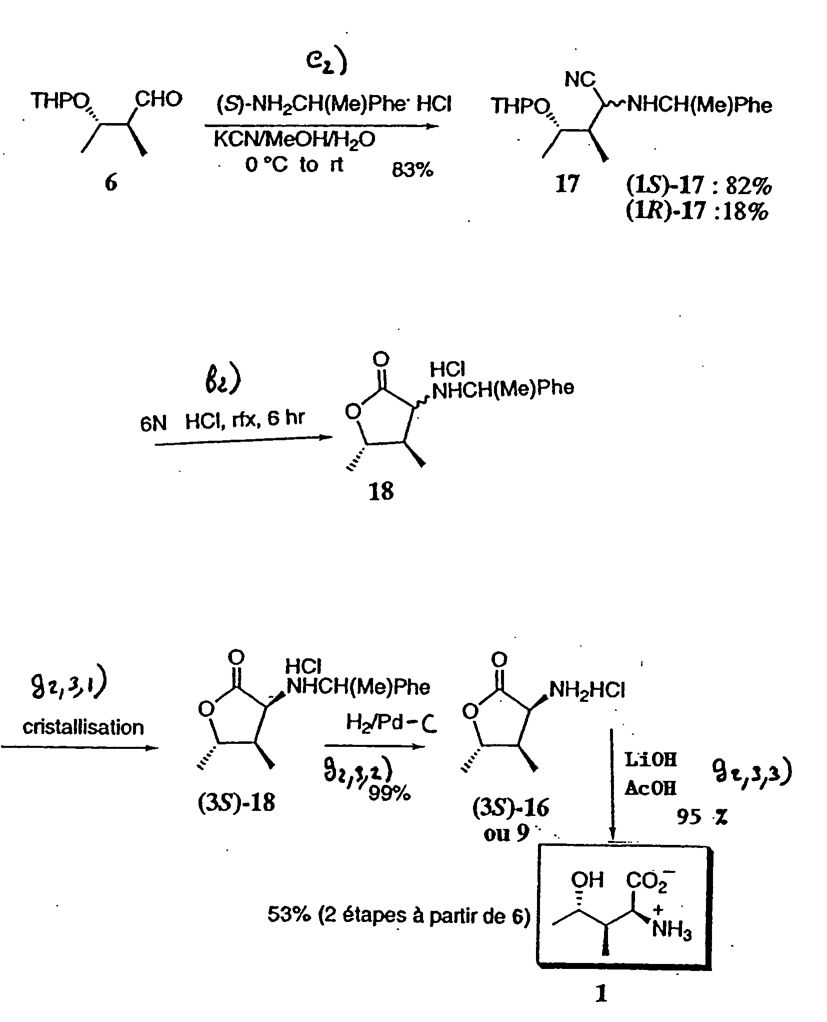

La présente invention concerne également les intermédiaires de synthèses. Ces derniers sont en particulier des lactones de formule générale **VIII** suivante : 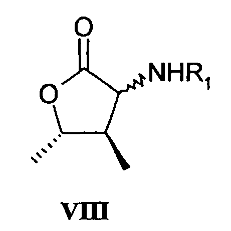 dans laquelle le groupe R₁ représente un groupe de formule -COOR₂ dans laquelle le groupe R₂ représente un groupe alkyle en C₁-C₆, un groupe aryle ou aralkyle.
Ces composés peuvent être présents sous forme libre ou sous forme de sels d'acides, en particulier minéraux du type HCl ou HBr.
Ils peuvent également être présents sous forme d'un mélange, en particulier racémique, des diastéréomères **(3S)** et **(3R)** ou sous leur forme optiquement pure (3S) ou **(3R).** En particulier dans le cas où le groupe R₁ représente le groupe -CH(CH₃)Ph, ces composés peuvent être présents sous forme d'un mélange de diastéréomères de rapport **(3S)/(3R)** de 4:1.

La présente invention concerne également les aminonitriles de formule générale **III** suivante : 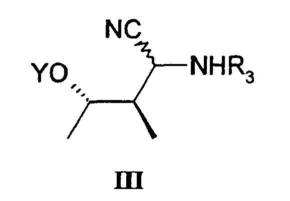 dans laquelle :
le groupe Y représente un groupe protecteur du groupe OH
et le groupe R₃ représente un groupe protecteur du groupe amine.
Ils peuvent être présents sous forme d'un mélange, en particulier racémique, des diastéréomères **(2S)** et **(2R)** ou sous leur forme optiquement pure **(2S)** ou (2R). En particulier dans le cas où le groupe R₃ représente le groupe -CH(CH₃)Ph, ces composés peuvent être présents sous forme d'un mélange de diastéréomères de rapport **(2S)/(2R)** de 4:1.
En particulier les aminonitriles selon la présente invention peuvent être représentés par la formule générale **V** suivante : 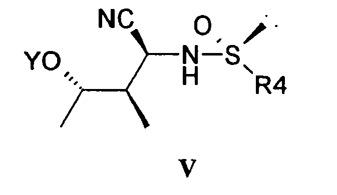 dans laquelle :
le groupe Y représente un groupe protecteur du groupe OH
et le groupe R4 représente un groupe alkyle en C₁-C₆, aryle, ou aralkyle.

La présente invention concerne également les composés de formule générale **IV** suivante : 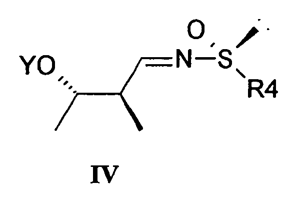 dans laquelle :
le groupe Y représente un groupe protecteur du groupe OH
et le groupe R4 représente un groupe alkyle en C₁-C₆, aryle ou aralkyle.

La présente invention concerne également les composés de formule générale **I** suivante : 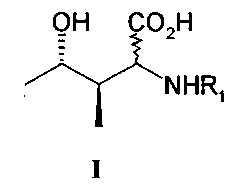 dans laquelle le groupe R₁ représente un groupe de formule -COOR₂ dans laquelle le groupe R₂ représente un groupe alkyle en C₁-C₆, un groupe aryle ou aralkyle. Ils peuvent être présents sous forme d'un mélange, en particulier racémique, des diastéréomères **(2S)** et **(2R)** ou sous leur forme optiquement pure **(2S)** ou **(2R).** En particulier dans le cas où le groupe R₁ représente le groupe -CH(CH₃)Ph, ces composés peuvent être présents sous forme d'un mélange de diastéréomères de rapport **(2S)/(2R)** de 4:1.

Les composés de formule générale **(2S)-1** dans laquelle le groupe R₁ représente un groupe protecteur du groupe aminé ou un groupe de formule -COOR₂ dans laquelle le groupe R₂ représente un groupe alkyle en C₁-C₆, un groupe aryle ou aralkyle peuvent être utiles en tant que médicament, en particulier destiné au traitement du diabète méllitusé non insulino-dépendant ou en tant qu'insulinotrope

Les compositions pharmaceutiques comprenant à titre de principe actif un composé de formule gént;ralc (2S)-1 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle et un excipient approprié peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques pour l'administration orale, sublinguale, sous-cutanée, intramusculaire, intraveineuse, transdermique, focale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale,

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

### Description des figures :

La figure 1 représente l'analyse GPC du mélange obtenu après incubation du composé **2** avec le *Geotrichum candidum.* Le gaz porteur est l'hélium avec une pression de 65 Kpa. La colonne utilisée est la DB.5 de dimensions 30 m X 0,32 mm. La détection se fait par détecteur à ionisation de flamme (FID) avec une isotherme à 60°C. Cette analyse permet de déterminer le pourcentage de conversion du composé **2** en composé **3** et l'excès diastéréomérique. A, B et C correspondent respectivement au composé **2,** au composé **3** et au (2R,3S)-2-méthyl-3-hydroxybutanoate d'éthyle.

La figure la représente l'analyse GPC du mélange après 30 heure d'incubation du composé **2** avec le *Geotrichum candidum.*

La figure 1b représente l'analyse GPC du mélange obtenu après 48 heures d'incubation du composé **2** avec le *Geotrichum candidum.*

La figure 2 représente les analyses HPLC lors de l'hydrolyse enzymatique de la lactone **11.** A et B correspondent respectivement à la **(3R)-(11)** et la **(3S)-(11).** Le solvant utilisé est un mélange de 35% d'acétonitrile et de 0,2% de triéthylamine dans l'eau. La détection se fait par UV à 250nm.

La figure 2a représente l'analyse HPLC analytique. La colonne utilisée est une C₁₈ hypersil ODS de dimensions, 5µm, 250 X 4,6mm. Le débit est de 1ml/min.

La figure 2b représente l'analyse HPLC préparative. La colonne utilisée est la prep nova-pak.HR C₁₈ de dimensions 6µm 60 A 10 X 2,5 cm. Le débit est de 6 ml/min.

La figure 3 a représente un dédoublement obtenu avec une poudre acétonique de pancréas de porc après 48 heures d'incubation. A et B correspondent respectivement à la **(3R)-(11)** et à la **(3S)-(11)**.

La figure 3b représente un dédoublement obtenu avec une souche microbienne *pénicillium* après 48 heures d'incubation. A et B correspondent respectivement à la **(3R)-(11)** et la **(3S)-(11).**

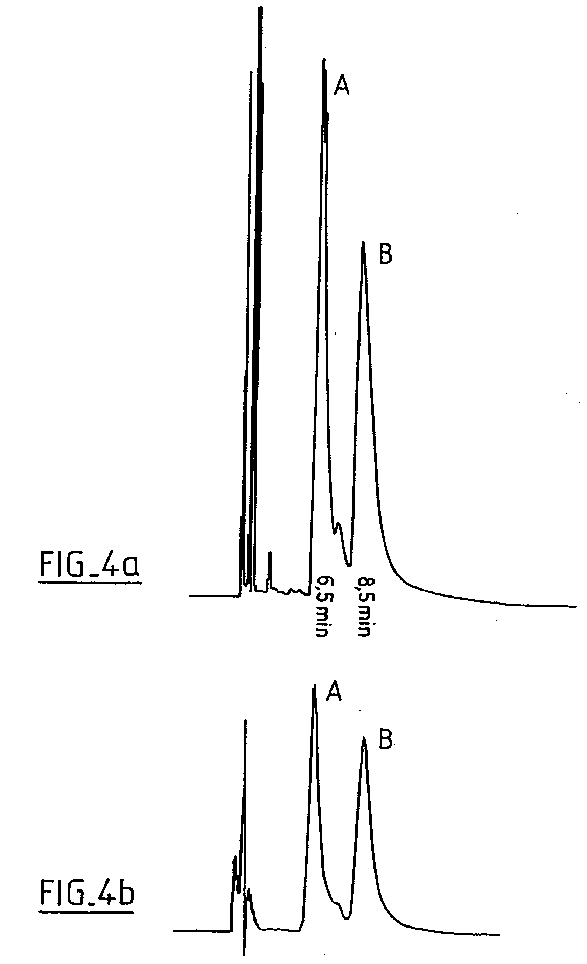

La figure 4 représente les analyses HPLC du mélange de diastéréomères de formule **N-Bn-1** lors du traitement avec le TFA. A et B correspondent respectivement à la **(2S)-N-Bn-1** et à la **(2R)-N-Bn-1.** Le solvant utilisé est un mélange de 20% d'acétonitrile et de 0,1% de TFA dans l'eau. La détection se fait par UV à 250nm.

La figure 4a représente l'analyse HPLC analytique à t = 0 heure. La colonne utilisée est une C₁₈ hypersil ODS de dimensions, 5µm, 250 X 4,6mm. Le débit est de 1ml/min.

La figure 4b représente l'analyse HPLC préparative. La colonne utilisée est la prep nova-pak.HR C₁₈ de dimensions 6µm 60 A 10 X 2,5 cm. Le débit est de 6 ml/min.

Les exemples de procédés de préparation suivant illustrent l'invention de façon non limitative.

### Le (2S,3S)-2-méthyl-3-hydroxybutanoate d'éthyle (3)

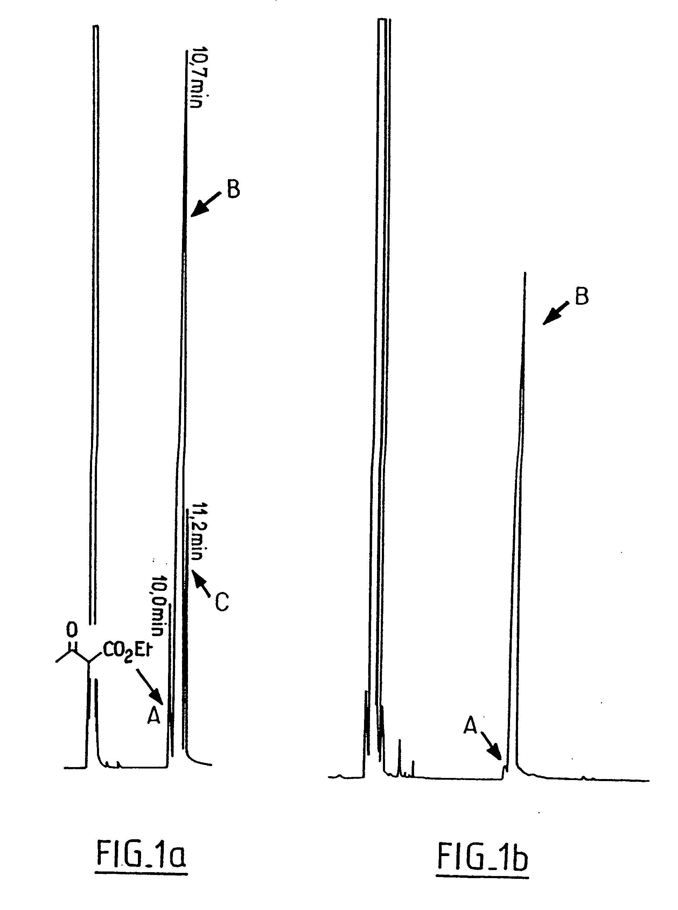

On a cultivé la souche *Geotrichum candidum* LCM dans un milieu stérile se composant (par litre d'eau distillée) de : KH₂PO₄ 1 g, K₂HPO₄ 2 g, MgSO₄ 0,5 g, FeSO₄ 20 mg, KCl 0,5 g, NaNO₃ 3 g, glucose 30 g, extrait soluble de maïs 10 g. On a placé la culture (6 litres) sur un agitateur rotatif pendant 3 jours (200 tr/mn, 27°C). Après croissance, on a récupéré le mycélium par filtration, on l'a remis en suspension dans de l'eau distillée (2 litres) et on a secoué pendant encore 24 heures (200 tr/mn, 27°C). On a filtré le mycélium et on l'a introduit dans le milieu réactionnel (2 litres) contenant du 2-méthylacétate d'éthyle **(2)** (10 à 20 g/l), du glucose à 1,5% et du NaCl à 1% (% par rapport au substrat). On a secoué le mélange (200 tr/mn, 27°C), on a prélevé des échantillons de 1 ml et on les a analysés de temps en temps par CPG afin de déterminer le pourcentage de conversion et l'excès diastéréomérique (de) (figure la : après 30 heures). Lorsque la réaction était complète (après environ 48 h : figure 1b), on a extrait le milieu avec de l'acétate d'éthyle afin d'obtenir le (2S,3S)-2-méthyl-3-hydroxybutanoate d'éthyle (3) avec un rendement de 90 à 95% : [α]_{D} = +26° (c 3,0; CHCl₃); RMN ¹H (250 MHz, CDCl₃) δ 4,18 (q, J = 7,1 Hz, 2H); 3,89 (quintuplet, J = 6,5 Hz, 1H); 2,64 (s large, 1H); 2,44 (quintuplet, J = 7,2 Hz, 1H); 1,28 (t, J = 7,1 Hz, 3H); 1,22 (d, J = 6,4 Hz, 3H); 1,19 (d, J = 7,2 Hz, 3H); RMN ¹³C (62,5 MHz, CDCl₃) δ 175,2; 68,6; 59,9; 46,7; 19,8; 13,7; 12,8; SM (CI) m/z 147 [M + H]⁺.

### Le (2S,3S)-anti-2-méthyl-3-tétrahydropyranyloxybutanoate d'éthyle (4)

### Préparation avec une résine échangeuse de cations (forme H+) :l'Amberlyst® H15 (commercialisée par Rohm et Haas)

De l'Amberlyst® H15 (162 mg) a été ajouté à une solution agitée de (2S,3S)-2-méthyl-3-hydroxybutanoate d'éthyle (3) (945 mg, 6,47 mmol) et de dihydropyranne (600 mg, 0,65 ml, 7,12 mmol) dans de l'heptane à 0°C et le mélange a été agité à 0°C pendant une heure puis à température ambiante pendant 6 heures. La résine a été enlevée par filtration et le solvant évaporé sous vide. Le résidu a été chromatographié sur gel de silice en utilisant comme éluant de l'heptane/EtOAc (20:1) pour donner le (2S,3S)-anti-2-méthyl-3-tétrahydropyranyloxybutanoate d'éthyle (4) (1,30 g, 87%).

### Préparation avec du para-toluène sulfonate de pyridine (PPTS)

On a agité pendant 24 heures à température ambiante une solution de (2S,3S)-2-méthyl-3-hydroxybutanoate d'éthyle (3) (5,245 g, 35,92 mmol) et de dihydropyranne (3,929 g, 4,26 ml, 46,70 mmol) dans du dichlorométhane sec contenant du PPTS (0,90 g, 3,59 mmol). On a éliminé le solvant sous vide et on a ajouté de l'éther. On a lavé la suspension avec de la saumure à demi saturée afin de retirer le catalyseur, puis on l'a lavée avec du NaHCO₃ saturé, de la saumure, on l'a séchée et évaporée. On a purifié le résidu au moyen d'une chromatographie sur colonne de gel de silice (heptane/EtOAc = 20:1) afin d'obtenir le (2S,3S)-anti-2-méthyl-3-tétrahydropyranyloxybutanoate d'éthyle (4) (8,18 g, 99%).

### Préparation par une quantité catalytique de l'acide para-toluènesulfonique monohydrate (TsOH·H₂O)

Du TsOH**·**H₂O (3,2 mg, 0,017 mmol, 0,2%) a été ajouté à température ambiante à une solution agitée de (2S,3S)-2-méthyl-3-hydroxybutanoate d'éthyle (3) (1,22 g, 8,36 mmol) et de dihydropyranne (773 mg, 0,84 ml, 9,19 mmol) dans du toluène (17 ml). Après agitation à la même température pendant 1 heure, la réaction a été stoppée par addition d'une solution aqueuse saturée de NaHCO₃. Le mélange a été extrait avec du toluène. La phase organique a été lavée avec de la saumure, séchée sur Na₂SO₄ et évaporée. Une chromatographie sur colonne de gel de silice (heptane/EtOAc = 20:1) a fourni le (2S,3S)-anti-2-méthyl-3-tétrahydropyranyloxybutanoate d'éthyle (4) (1,91 g, 99%) : IR (CHCl₃) 3009, 2982, 2945, 2872, 1727, 1455, 1381, 1324, 1262, 1235, 1190 cm⁻¹; RMN ¹H (250 MHz, CDCl₃) δ 4,80 à 4,77 (m, 0,5H); 4,65 à 4,62 (m, 0,5H); 4,21 à 3,77 (m, 4H); 3,56 à 3,45 (m, 1H); 2,71 à 2,53 (m, 1H); 1,81 à 1,47 (m, 6H); 1,28 (t, J = 7,2 Hz, 1,5H); 1,26 (t, J = 7,1 Hz, 1,5H); 1,24 (d, J =6,2 Hz, 1,5H); 1,15 (d, J = 7,1 Hz, 1,5H); 1,12 (d, J = 6,2 Hz, 1,5H); 1,10 (d, J = 7,1 Hz, 1,5H); RMN ¹³C (62,5 MHz, CDCl₃) δ 174,2; 99,4; 94,4; 75,8; 71,6; 62,1; 61,2; 59,6; 45,5; 30,7; 30,5; 25,1; 19,4; 18,7; 18,1; 14,9; 13,8; 12,0; 11,9; SM (CI) m/z 231 [M + H]⁺. Analyse : Calculé pour C₁₂H₂₂O₄: C, 62,58; H, 9,63; Trouvé : C, 62, 71; H, 9, 67.

### Le (2R,3S)-2-méthyl-3-tétrahydropyranyloxybutanol (5)

A une suspension agitée de LiAlH₄ (2,70 g, 71,12 mmol) dans du THF/toluène (2 : 1) (178 ml), on a ajouté, goutte à goutte, une solution de (2S,3S)-2-méthyl-3-tétrahydropyranyloxybutanoate d'éthyle (4) (8,18 g, 35,56 mmol) à 0°C. On a agité le mélange réactionnel à 0°C pendant 30 minutes, puis à température ambiante pendant 30 minutes. On a neutralisé la réaction au moyen du "traitement Fieser"; on a traité la réaction en ajoutant successivement goutte à goutte, 2,7 ml d'eau glacée, 2,7 ml d'une solution aqueuse de NaOH à 15%, et 3 x 2,7 ml d'eau glacée. Après avoir agité pendant au moins 30 minutes, on a filtré le mélange à travers de la Célite. Ensuite on a lavé les sels d'aluminium précipités avec de l'EtOAc. On a évaporé le solvant, on a purifié l'huile résiduelle au moyen d'une chromatographie éclair sur colonne de gel de silice (heptane/EtOAc = 5:1 puis 2:1) afin d'obtenir le (2R,3S)-2-méthyl-3-tétra-hydropyranyloxybutanol (5) sous forme d'une huile incolore (6,59 g, 99%) : RMN ¹H (250 MHz, CDCl₃) δ 4,69 à 4,67 (m, 0,5H); 4,58 à 4,56 (m, 0,5H); 3,98 à 3,43 (m, 5H); 1,80 à 1,48 (m, 7H); 1,29 (d, J = 6,3 Hz, 1, 5H); 1,18 (d, J = 6,1 Hz, 1,5 H); 0,95 (d, J = 7,0 Hz, 1,5H); 0,94 (d, J = 7,0 Hz, 1,5H); RMN ¹³C (50 MHz, CDCl₃) δ 99,9; 97,4; 78,4; 74,9; 65,2; 64,2; 62,7; 41,2; 40,6; 31,3; 31,0; 25,3; 25,1; 20,7; 19,8; 18,9; 17,5; 14,0; 13,0; SM (CI) m/z 189 [M + H]⁺. SMHR calculé pour C₁₀H₂₁O₃ (M + H), 189,14906; trouvé, 189,14755.

### La (2S,3S)-2-méthyl-3-tétrahydropyranyloxybutyraldéhyde (6)

### Oxydation par du TEMPO

A un mélange biphasique froid (0°C), rapidement agité (> 1000 tr/mn), constitué de (2R,3S)-2-méthyl-3-tétrahydropyranyloxybutanol (5) (1,88g, 10 mmol), de TEMPO à radicaux libres (31,25 mg, 2%), de bromure de sodium (1,029g, 10 mmol), de toluène (30 ml), d'EtOAc (30 ml) et d'H₂O (5 ml), on a ajouté goutte à goutte une solution aqueuse de NaOCl (11 mmol, 5,3 ml d'une solution à 12,5%) et du NaHCO₃ (2,43 g, 29 mmol) sur une période de 1 heure. La phase aqueuse a été séparée et lavée avec de l'Et₂O (50 ml). Les phases organiques réunies ont été lavées avec une solution de KI (80 mg) dissoute dans du KHSO₄ aqueux à 10% (20 ml), puis avec une solution aqueuse à 10% de thiosulfate de sodium (10 ml), de la saumure (20 ml), et séchées (MgSO₄). La filtration et la concentration sous vide ont fourni l'aldéhyde 6 désiré (1,72 g, 92%) qui a été utilisé pour les réactions suivantes sans autre purification.

### Oxydation par Py·SO₃/DMSO

A une solution de (2R,3S)-2-méthyl-3-tétrahydropyranyloxybutanol (5) (1,39 g, 7,39 mmol) et d'Et₃N anhydre (4,94 g, 6,78 ml, 48,77 mmol) dans du DMSO (25 ml), on a ajouté en portions le complexe Py**·**SO₃ (7,06 g, 44,36 mmol) à 0°C. On a agité le mélange réactionnel à 0°C pendant 3 heures, puis à température ambiante pendant 1 heure, puis on l'a divisé entre de l'eau et de l'éther. On a extrait la couche aqueuse avec de l'éther. On a lavé les extraits éthérés réunis avec du HCl 1M, de l'eau, du NaHCO₃ saturé, de la saumure, on les a séchés sur Na₂SO₄ et évaporés afin d'obtenir le (2S,3S)-2-méthyl-3-tétrahydropyranyloxybutyraldéhyde brut (6) (1,39 g, 100%) que l'on a utilisé directement pour la réaction suivante sans purification : RMN ¹H (250 MHz, CDCl₃) δ 9,78 (d, J = 2,7 Hz, 0,5H); 9,74 (d, J = 2,0 Hz, 0,5H); 4,76 à 4,62 (m, 1H); 4,15 à 3,73 (m, 2H); 3,53 à 3,43 (m, 1H); 2,62 à 2,46 (m, 1H); 1,82 à 1,51 (m, 6H); 1,29 (d, J = 6,2 Hz, 1,5H); 1,18 (d, J = 6,2 Hz, 1,5H); 1,11 (d, J = 7,2 Hz, 1,5H); 1,07 (d, J = 7,1 Hz, I,5H); RMN ¹³C (62,5 MHz, CDCl₃) δ 203,5; 203,1; 99,0; 95,1; 74,4; 71,0; 62,0; 51,6; 51,1; 30,5; 25,0; 19,2; 19,1; 18,6; 16,0; 9,7; 9,1; SM (CI) m/z 187 [M + H]⁺.

### La (S)-N-[(2R,3S)-2-méthyl-3-tétrahydropyranyloxybutylidène]-p-toluène-sulfinamide (7)

A un mélange de (S)-(+)-p-toluènesulfinamide (233 mg, 1,50 mmol) et de (2S,3S)-2-méthyl-3-benzyloxybutyraldéhyde (6) (280 mg, 1,50 mmol) dans du dichlorométhane (25 ml), on a ajouté, goutte à goutte, du Ti(OEt)₄ (1,71 g, 1,57 ml, 7,5 mmol, 5 éq.) à température ambiante. On a mis la solution à reflux sous argon pendant 5,5 heures. On a neutralisé la réaction à 0°C en ajoutant de l'eau (25 ml). On a filtré la solution trouble à travers de la Célite, et on a lavé le gâteau de filtration avec du dichlorométhane (2 x 25 ml). On a séparé les phases, on a extrait la phase aqueuse avec du dichlorométhane et on a séché les portions organiques réunies sur Na₂SO₄ et on les a concentrées. Une chromatographie éclair sur colonne de gel de silice (heptane/EtOAc = 6:1) a produit du (S)-N-[(2R,3S)-2-méthyl-3-tétrahydropyranyloxybutylidène]-p-toluènesulfinamide (7) (375 mg, 77%): IR (CHCl₃) 3010, 2976, 2946, 2878, 2854, 1620, 1598, 1494, 1455, 1443, 1380, 1354, 1214 cm⁻¹; RMN ¹H (250 MHz, CDCl₃) δ 8,31 (d, J = 5,8 Hz, 0,5H); 8,23 (d, J = 5,3 Hz, 0,5H); 7,55 (m, 2H); 7,29 (m, 2H); 4,79 à 4,70 (m, 0,5H); 4,55 à 4,51 (m, 0,5H); 4,00 à 3,82 (m, 1,5H); 3,77 à 3,70 (m, 0,5H); 3,48 à 3,41 (m, 1H); 2,84 à 2,64 (m, 1H); 2,39 (s, 3H); 1,78 à 1,41 (m, 6H); 1,25 (d, J = 6,4 Hz, 1,SH); 1,14 (d, J = 5,7 Hz, 3H); 1,14 (d, J = 7,1 Hz, 1, 5H); 1,09 (d, J = 7,0 Hz, 1,5H); RMN ¹³C (75 MHz, CDCl₃) δ 169,2; 168,6; 141,9; 141,8; 141,2; 129,4; 124,4; 124,2; 99,6; 94,6; 76,4; 72,2; 62,3; 61,8; 45,6; 45,2; 30,6; 30,5; 25,2; 25,2; 21,1; 19,4; 19,1; 18,9; 16,3; 13,0; 12,5; SM (CI) m/z 324 [M + H]⁺. SMHR : Calculé pour C₁₇H₂₆NO₃S (M + H), 324,16333; trouvé, 324,16141.

### La (S)-N-[(1S,2R,3S)-1-cyano-2-méthyl-3-tétrahydropyranyloxybutyl]-p-toluène-sulfinamide (8)

A une solution d'isopropanol (56 mg, 72 ml, 0,94 mmol, 1,1 éq.) dans du THF, on a ajouté de l'Et₂AlCN (1,0 M dans du toluène, 1,28 ml, 1,28 mmol, 1,5 éq.), et on a agité la solution à température ambiante pendant 15 minutes.

A une solution de (S)-N-[(2R,3S)-2-méthyl-3-tétrahydropyranyloxybutylidène]-p-toluènesulfinamide (7) (275 mg, 0,85 mmol) dans du tétrahydrofuranne (THF), on a ajouté la solution d'Et(OiPr)AlCN préparée ci-dessus dans du THF à -70°C. Après 15 minutes, on a amené le mélange réactionnel à température ambiante, on a agité et contrôlé la disparition du sulfinamide (22 heures) au moyen d'une CCM. On a refroidi le mélange réactionnel à -70°C, on l'a neutralisé en ajoutant du NaHCO₃ aqueux. On a dilué la suspension avec de l'EtOAc, on l'a filtrée à travers de la Célite et on l'a diluée avec de l'eau et on a extrait la phase aqueuse avec de l'EtOAc (2 x). On a lavé les phases organiques réunies avec de la saumure, on les a séchées et évaporées. Une chromatographie éclair sur colonne (SiO₂, heptane/EtOAc = 5:1 puis 3:1) a produit du (S)-N-[(1S,2R,3S)-1-cyano-2-méthyl-3-tétrahydro-pyranyloxybutyl]-p-toluènesulfinamide (**8**) (283 mg, 95%) : IR (CHCl₃) 3672, 3368, 3252, 3012, 2948, 2857, 2243, 1598, 1492, 1454, 1380, 1343, 1236, 1174 cm⁻¹; RMN ¹H (250 MHz, CDCl₃) δ 7,65 à 7,59 (m, 2H); 7,36 à 7,27 (m, 2H); 6,16 (d, J = 9,6 Hz, 0,5H); 5,11 (m, 0,5H); 4,60 à 4,49 (m, 1,5H); 4,16 (dd, J = 9,6, 2,6 Hz, 5H); 3,94 à 3,85 (m, 0,5H); 3,75 à 3,61 (m, 1H); 3,57 à 3,34 (m, 1,5H); 2,42 (s, 3H); 2,14 à 1,96 (m, 1H); 1,89 à 1,37 (m, 6H); 1,33 (d, J = 6,2 Hz, 1,5H); 1,18 (d, J = 6,1 Hz, 3H); 1,07 (d, J = 6,9 Hz, 1,5H); 1,06 (d, J = 6,9 Hz, 1,5H); RMN ¹³C (62,5 MHz, CDCl₃) δ 141,7; 141,5; 139,8; 139,4; 129,6; 125,8; 118,5; 117,5; 100,5; 96,7; 77,6; 72,1; 64,0; 62,3; 45,5; 44,4; 43,9; 30,9; 30,6; 25,1; 24,9; 21,1; 20,4; 19,3; 19,3; 16,9; 13,3; 11,6; SM (CI) m/z 351 [M + H]⁺. SMHR : Calculé pour C₁₈H₂₇N₂O₃S (M + H), 351,17423; trouvé, 351,17621.

### Le (35,4R,5S)-3-amino-4 méthyl-5-méthyl-2-oxo-tétrghydrofuranne (9)

On a mis à reflux une solution de (S)-N-[(1S,2R,3S)-1-cyano-2-méthyl-3-tétrahydropyranyloxybutyl]-p-toluènesulfinamide (**8**) (200 mg, 0,57 mmol) dans du HCl 6N (14 ml) pendant 6 heures. On a lavé le mélange réactionnel avec du dichlorométhane, et on l'a évaporé afin d'obtenir le chlorhydrate de (3S,4R,5S)-3-amino-4-méthyl-5-méthyl-2-oxo-tétrahydrofuranne (9) (94 mg, 100%). [α]_{D}= -18° (c 1,4; MeOH); IR (nujol) 3526, 2924, 2854, 1771, 1581, 1504, 1462, 1385, 1355, 1315, 1260, 1209 cm⁻¹; RMN ¹H (250 MHz, D₂O) δ 4,61 à 4,54 (m, 2H); 2,72 (quintuplet large, J = 7,5 Hz, 1H); 1,40 (d, J = 6,6 Hz, 3H); 1,11 (d, J = 7,3 Hz, 3H); RMN ¹³C (50 MHz, D₂O) δ 174,3; 85,2; 52,3; 38,1; 19,5; 13,0; SM (CI) m/z 130 [M + H]⁺. SMHR: Calculé pour C₆H₁₂NO₂ (M + H), 130,08680; trouvé, 130,08604.

### La (2S,3R,4S)-4-hydroxyisoleucine (1) à partir de (3S)-16 ou de (9)

### Premier procédé :

A une solution de chlorhydrate de (3S,4R,5S)-3-amino-4-méthyl-5-méthyl-2-oxo-tétrahydrofuranne (9) (28 mg, 0,17 mmol) dans un mélange de THF/H₂O/méthanol (MeOH) (1:1:10, 3 ml), on a ajouté du LiOH-H₂O (16 mg, 0,37 mmol). On a agité la solution à température ambiante pendant 4 heures. On a évaporé le solvant. On a dissout le résidu dans de l'eau , puis on l'a fait passer à travers une colonne de résine échangeuse d'ions Dowex 50WX8 (forme H⁺). On a lavé minutieusement la colonne avec de l'eau et on a élué l'acide aminé avec du NH₄OH 2M afin d'obtenir la (2S,3R,4S)-4-hydroxyisoleucine (**1**) (16 mg, 70%)

### Deuxième procédé :

A une solution de chlorhydrate de (3S,4R,5S)-3-amino-4-méthyl-5-méthyl-2-oxo-tétrahydrofuranne (9) (8,30 g, 50,3 mmol) ou de (3S,4R,5S)-3-amino-4-méthyl-5-méthyl-2-oxo-tétrahydrofuranne (3S)-(16) dans de l'H₂O (250 ml), on a ajouté du LiOH-H₂O (4,22 g, 100,6 mmol). On a agité la solution à température ambiante pendant 18 heures. Après addition de l'acide acétique (2,9 ml, 50,3 mmol) on a évaporé le solvant jusqu'au séchage complet à température ambiante par évaporateur rotatif Le résidu a été cristallisé à partir d'éthanol à 90% de façon à d'obtenir la (2S,3R,4S)-4-hydroxyisoleucine (1) (6,65 g, 90 %) : RMN ¹H (250 MHz, D₂O) δ 3,84 (d, J = 4,4 Hz, 1 H); 3,78 (m, 1H); 1,87 (m, 1H); 1,19 (d, J = 6,4 Hz, 3H); 0,91 (d, J = 7,0 Hz, 3 H); RMN ¹³C (50 MHz, D₂O) δ 174,5; 70,6; 57,7; 42,0; 21,4; 12,8; SM (CI) m/z (sel d'HCl) 184 [M + H]⁺. Microanalyse : Calculé pour C₆H₁₃NO₃ C, 48,97; H, 8,90; N, 9,52; trouvé, C, 49,04; H, 8,83; N, 9,60.

### La (2R,3S)-1-cyano-2-méthyl-3-tétrahydropyranyloxy-N-benzylbutylamine (10)

### Procédé 1

A une solution de l'aldéhyde 6 préparé ci-dessus (500 mg, 2,69 mmol) dans du dichlorométhane (9 ml), on a ajouté de la benzylamine (346 mg, 353 ml, 3,23 mmol) à température ambiante et on a poursuivi l'agitation à température ambiante pendant 2 heures. On a refroidi le mélange réactionnel à 0°C, on a introduit successivement du méthanol (3 ml) et du TMSCN (400 mg, 538 ml, 4,03 mmol). Après agitation à 0°C pendant 2 heures puis à température ambiante pendant 22 heures, on a séparé le mélange réactionnel dans du NaHCO₃ saturé et de l'éther. On a extrait la phase aqueuse avec de l'éther, on a lavé les extraits éthérés avec de la saumure, on les a séchés et filtrés à travers un disque court de Célite, et du Na₂SO₄, puis évaporés. Une chromatographie éclair sur colonne de gel de silice avec pour éluant, de l'heptane/EtOAc = 10:1, a produit de la (1R,2R,3S)-1-cyano-2-méthyl-3-tétrahydropyranyloxy-N-benzylbutylamine **(1R)-(10)** et de la (1S,2R,3S)-1-cyano-2-méthyl-3-tétrahydropyranyloxy-N-benzylbutylamine **(1S)-(10)** (au total 735 mg, 90%).

*La (1R,2R,4S)-1-cyano-2-méthyl-3-tétrahydropyranyloxy-N-benzyl-butylamine (1R)-(10)*
IR (CHCl₃) 3510, 3338, 3013, 2946, 2854, 1605, 1496, 1455, 1386, 1355, 1276, 1232 cm⁻¹; RMN ¹H (250 MHz, CDCl₃) δ 7,39 à 7,26 (m, 5H); 4,66 à 4,64 (m, 0,5H); 4,58 à 4,55 (m, 0,5H); 4,05 (d, J = 12,5 Hz, 1H); 3,99 à 3,65 (m, 4H); 3,51 à 3,43 (m, 1H); 2,06 à 1,92 (m, 1H); 1,78 à 1,38 (m, 6H); 1,28 (d, J = 6,2 Hz, 1,5H); 1,14 (d, J = 6,2 Hz ,1,5H); 1,07 (d, J = 7,1 Hz, 1,5H); 1,04 (d, J = 6,8 Hz, 1,5H); RMN ¹³C (62,5 MHz, CDCl₃) δ 138,8, 138,3; 128,4; 128,3; 128,2; 127,2; 127,0; 119,8; 119,7; 100,8; 95,9;77,6; 71,8; 63,4; 62,4; 52,4; 52,1; 51,7; 42,8; 42,2; 31,0; 30,7; 25,1; 20,2; 19,8; 19,6; 16,8; 12,8; 12,7; SM (CI) m/z 303 [M + H]⁺. SMHR : Calculé pour C₁₈H₂₇N₂O₂ (M + H), 303,20724; trouvé, 303,20621.

*La (1S,2R,3S)-1-cyano-2-méthyl-3-tétrahydropyranyloxy-N-benzyl-butylamine **(1S)-(10)***
IR (CHCl₃) 3504, 3340, 3013, 2947, 2854, 1605, 1497, 1455, 1384, 1354, 1262, 1231 cm⁻¹; RMN ¹H (250 MHz, CDCl₃) δ 7,35 à 7,26 (m, 5H); 4,60 (m, 1H); 4,12 à 4,05 (m, 1H); 3,94 à 3,58 (m, 4H); 3,52 à 3,40 (m, 1H); 2,03 à 1,89 (m, 1H); 1,79 à 1,46 (m, 6H), 1,25 (d, J = 6,2 Hz, 1,5H); 1,11 (d, J = 6,2 Hz, 1,5H); 1,05 (d, J = 6,7 Hz, 1,5H); 1,03 (d, J = 6,8 Hz, 1,5H); RMN ¹³C (62,5 MHz, CDCl₃) δ 138,6; 138,2; 128,3; 127,4; 127,2; 119,6; 119,1; 100,4; 96,2; 76,6; 71,7; 63,4; 62,9; 51,8; 51,5; 42,5; 42,3; 31,0; 30,9; 25,2; 20,1; 19,8; 18,7; 16,3; 12,7; 11,6; SM (CI) m/z 303 [M + H]⁺. SMHR : Calculé pour C₁₈H₂₇N₂O₂ (M + H), 303,20724; trouvé, 303,20876.

### La (2R,3S)-1-cyano-2-méthyl-3-tétrahydropyranyloxy-N-benzylbutylamine (10)

### Procédé 2

A une solution de l'aldéhyde 6 préparé ci-dessus (1,58 g, 8,49 mmol) et de chlorhydrate de benzylamine (1,21 g, 8,49 mmol) dans du méthanol (20 ml) et de l'eau (20 ml), on a ajouté du KCN (553 mg, 8,49 mmol) à température ambiante. Après agitation à température ambiante pendant 24 heures, on a séparé le mélange réactionnel dans du NaHCO₃ saturé et de l'éther. On a extrait la phase aqueuse avec de l'éther, on a lavé les extraits éthérés avec de la saumure, on les a séchés et filtrés à travers un disque court de Célite et Na₂SO₄ puis évaporés. Une chromatographie éclair sur colonne de gel de silice avec pour éluant de l'heptane/EtOAc = 10:1 a produit la (1R,2R,3S)-1-cyano-2-méthyl-3-tétrahydropyranyloxy-N-benzyl-butylamine **(1R)-(10)** et la (1S,2R,3S)-1-cyano-2-méthyl-3-tétrahydropyranyloxy-N-benzylbutylamine **(1S)-(10)** (au total 2,097 g, 82%).

### Le (3R,4R,5S)-3-N-benzylamino-4-méthyl-5-méthyl-2-oxo-tétrahydrofuranne (3R)-(11) et le (3S,4R,5S)-3-N-benzylamino-4-méthyl-5-méthyl-2-oxo-tétrahydrofuranne (3S)-(11) à partir de la (1R,2R,3S)-1-cyano-2-méthyl-3-tétrahydropyranyloxy-N-benzylbutylamine (1R)-(10) et de la (1S,2R,3S)-1-cyano-2-méthyl-3-tétrahydro-pyranyloxy-N-benzyl-butylamine (1S)-(10)

On a mis à reflux pendant 6 heures une solution de la (1R,2R,3S)-1-cyano-2-méthyl-3-tétrahydropyranyloxy-N-benzylbutylamine **(1R)-(10)** et de la (1S,2R,3S)-1-cyano-2-méthyl-3-tétrahydropyranyloxy-N-benzylbutylamine **(1S)-(10)** (375 mg, 1,24 mmol) dans du HCl 6N (25 ml). On a lavé le mélange réactionnel avec du dichlorométhane et on l'a évaporé afin d'obtenir le chlorhydrate de (3R,4R,5S)-3-N-benzylamino-4-méthyl-5-méthyl-2-oxo-tétrahydrofuranne **(3R)-(11)** et le chlorhydrate de (3S,4R,5S)-3-N-benzylamino-4-méthyl-5-méthyl-2-oxo-tétrahydrofuranne **(3S)-(11)** (316 mg, 100%) dans un rapport de 45:55.

### Le (3S,4R,5S)-3-N-benzylamino-4-méthyl-5-méthyl-2-oxo-tétrahydrofuranne (3S)-(11) à partir de la (1S,2R,3S)-1-cyano-2-méthyl-3-tétrahydropyranyloxy-N-benzyl-butylamine (1S)-(10)

On a mis à reflux pendant 6 heures une solution de la (1S,2R,3S)-1-cyano-2-méthyl-3-tétrahydropyranyloxy-N-benzylbutylamine **(1S)-10** (34 mg, 0,11 mmol) dans du HCl 6N (2 ml). On a lavé le mélange réactionnel avec du dichlorométhane, on l'a basifié avec du NaHCO₃ aqueux saturé, on l'a extrait avec de l'EtOAc. On a lavé les extraits d'EtOAc avec de la saumure, on les a séchés et évaporés pour obtenir le (3S,4R, 5 S)-3-N-benzylamino-4-méthyl-5-méthyl-2-oxo-tétrahydrofuranne (une γ-lactone) **(3S)-(11)** (24 mg, 100%) : [α]_{D} = -14° (c 2,2, CHCl₃); IR (CHCl₃) 3528, 3333, 3088, 3066, 3029, 2981, 2935, 2875, 2822, 1770, 1605, 1496, 1455, 1384, 1361, 1300, 1229, 1178 cm⁻¹ ; RMN ¹H (250 MHz, CDCl₃) δ 7,38 à 7,18 (m, 5H); 4,31 (qd, J = 6,5, 3,5 Hz, 1H); 3,93 (d, J = 13,2 Hz, 1H); 3,86 (d, J = 13,2 Hz, 1H); 3,61 (d, J = 7,3 Hz, 1H); 2,26 (quintuplet-d, J = 7,1, 3,4 Hz, 1H); 1,75 (s large, 1H, NH), 1,36 (d, J = 6,5 Hz, 3H); 1,07 (d, J = 7,1 Hz, 3H); RMN ¹³C (62,5 MHz, CDCl₃) δ 176,5; 139,3; 128,4; 128,1; 127,2; 81,5; 58,1; 52,1; 40,1; 19,6; 12,6; SM (CI) m/z (sel de HCl) 220 [M + H]⁺. Le sel de HCl : RMN ¹H (250 MHz, D₂O) δ 7,52 (m, 5H); 4,70 (d, J = 7,7 Hz, 1H); 4,64 (m, 1H); 4,60 (d, J = 13,1 Hz, 1H); 4,42 (d, J = 13,1 Hz, 1H); 2,84 (quintuplet, J = 7,2, 1H); 1,42 (d, J = 6,7 Hz, 3H), 1,18 (d, J = 7,2 Hz, 3H); RMN ¹³C (62,5 MHz, D₂O+CD₃OD) δ 173,1; 131,2; 131,0; 130,3; 84,9; 58,1; 52,0; 38,3; 19,5; 13,8; SMHR : Calculé pour C₁₃H₁₈NO₂ (M + H), 220,13374; trouvé, 220,13562.

### Le (3R,4R,5S)-3-N-benzylamino-4-méthyl-5-méthyl-2-oxo-tétrahydrofuranne (3R)-(11) à partir de la (1R,2R,3S)-1-cyano-2-méthyl-3-tétrahydropyranyloxy-N-benzyl-butylamine (1R)-(10)

On a mis à reflux pendant 6 heures une solution de la (1R,2R,3S)-1-cyano-2-méthyl-3-tétrahydropyranyloxy-N-benzylbutylamine **(1R)-10** (20 mg, 0,066 mmol) dans du HCl 6N (1,5 ml). On a lavé le mélange réactionnel avec du dichlorométhane, on l'a basifié avec du NaHCO₃ aqueux saturé, on l'a extrait avec de l'EtOAc. On a lavé les extraits d'EtOAc avec de la saumure, on les a séchés et évaporés pour obtenir le (3R,4R,5S)-3-N-benzylamino-4-méthyl-5-méthyl-2-oxo-tétrahydrofuranne (une γ-lactone) **(3R)-(11)** (13,5 mg, 93%) : [α]_{D} = +48° (c 2,4, CHCl₃); IR (CHCl₃) 3528, 3327, 3030, 2980, 2934, 2913, 2877, 1770, 1604, 1455, 1389, 1328, 1231, 1224, 1211, 1187 cm⁻¹; RMN ¹H (250 MHz, CDCl₃) δ 7,39 à 7,23 (m, 5H); 4,06 à 3,94 (m, 3H); 3,18 (d, J = 11,4 Hz, 1H); 2,01 à 1,85 (m, 2H); 1,39 (d, J = 6,1 Hz, 3H); 1,14 (d, J = 6,5 Hz, 3H); RMN ¹³C (62,5 MHz, CDCl₃) δ 177,0; 139,6; 128,3; 128,0; 127,0; 79,5; 63,4; 51,3; 45,4; 18,4; 14,3; SM (CI) m/z (sel de HCl) 220 [M + H]⁺. Le sel de HCl : RMN ¹H (250 MHz, D₂O) δ 7,50 (m, 5H); 4,56 (d, J = 12,9 Hz, 1H); 4,45 à 4,34 (m, 1H); 4,39 (d, J = 12,9 Hz, 1H); 4,26 (d, J = 11,5 Hz, 1H); 2,58 à 2,41 (m, 1H); 1,46 (d, J = 6,2 Hz, 3H), 1,25 (d, J = 6,5 Hz, 3H); RMN ¹³C (62,5 MHz, D₂O) δ 173,0; 130,6; 130,4; 129,9; 82,6; 67,0; 61,4; 50,3; 42,0; 17,9; 13,6; SMHR : Calculé pour C₁₃H₁₈NO₂ (M + H), 220,13374; trouvé, 220,13195.

### La (2S,3R,4S)-N-benzyl-4-hydroxyisoleucine (2S)-N-Bn-1 par hydrolyse alcaline du (3S)-11

Une solution de lactone **(3S)-11** (55 mg, 0,25 mmol) dans de l'H₂O (2 ml) contenant du LiOH·H₂O (12 mg, 0,27 mmol) a été agitée pendant 2 heures à température ambiante. Après addition d'AcOH (16µl, 0,27 mmol), la solution a été extraite avec de l'EtOAc (3 X 5 ml), séchée sur Na₂SO₄ et le solvant a été évaporé pour donner la (25,3R4S)-N-Bn-4-hydroxyisoleucine **(2S)-N-Bn-1** (55 mg, 93%) : [α]_{D} = -5° (c 0,9, H₂O); RMN ¹H (250 MHz, D₂O) δ 7,31 (5H); 4,13 (d, J = 13,0 Hz, 1H); 3,95 (d, J = 13,0 Hz, 1H); 3,53 (m, 2H); 1,68 (m, 1H); 1,01 (d, J = 6,3 Hz, 3H); 0,95 (d, J = 7,0 Hz, 3H); RMN ¹³C (50 MHz, D₂O) δ 174,2; 131,2; 130,6; 130,3; 129,9; 71,3; 65,0; 51,2; 41,6; 21,4; 12,7.

### La (2R,3R,4S)-N-benzyl-4-hydroxyisoleucine (2R)-N-Bn-1 par hydrolyse alcaline du (3R)-11

La **(3R)-11** (197 mg, 0,9 mmol) a été hydrolysée de la même façon que décrit ci-dessus pour donner la (2R,3R,4S)-N-benzyl-4-hydroxyisoleucine **(2R)-N-Bn-1** (205 mg, 96%) :Point de fusion = 193-194°C (décomposition); [α]_{D} = +24° (c 1, H₂O); RMN ¹H (250 MHz, D₂O) δ 7,48 (5H); 4,35 (d, J = 13,1 Hz, 1H); 4,07 (d, J = 13,1 Hz, 1H); 3,82 (m, 2H); 2,02 (m, 1H); 1,00 (d, J = 6,1 Hz, 3H); 0,99 (d, J = 7,1 Hz, 3H); RMN ¹³C (50 MHz, D₂O) δ 174,7; 131,7; 130,8; 130,3; 129,9; 70,9; 62,2; 51,4; 40,1; 20,8; 12,7.

### Cyclisation de la (2R)-N-Bn-1 en lactone (3R)-11

On a ajouté de l'acide trifluoroacétique (200 µl) à une solution de **(2R)-N-Bn-1** (40 mg) dans de l'H₂O (10 ml) et on l'a chauffée à 45°C pendant 5 minutes. Le solvant a été évaporé par rotavapeur à 45°C. Le résidu a été dissout dans de l'EtOAc (10 ml), lavé avec une solution saturée de NaHCO₃, de la saumure et séché sur Na₂SO₄ pour donner la lactone **(3R)-11** (33 mg, 89%).

### La (2S,3R,4S)-4-hydroxyisoleucine (1) à partir de (11)

On a agité une solution de 11 (500 mg, 2,28 mmol) dans de l'H₂O (20 ml) contenant du LiOH·H₂O (100 mg) pendant 2 heures à température ambiante. La fin de l'hydrolyse a été confirmée par CCM (EtOAc/heptane = 1:3). On a ajouté à cette solution du TFA (0,4 ml) et le solvant a été évaporé par rotavapeur à 50°C. Le résidu a été dissout dans de l'H₂O. L'analyse HPLC a démontré la disparition totale du pic correspondant à la **(2R)-N-Bn-1** et la réapparition de la **(3R)-11**. La solution ci-dessus a été extraite avec de l'EtOAc pour enlever la lactone **(3R)-11** (63 mg, 38%) et la phase aqueuse a été traitée avec du H₂ en présence de 10% de palladium sur du carbone (10 mg) pendant 6 heures à température ambiante. Après filtration du catalyseur, le solvant a été condensé et passé à travers une résine échangeuse d'ions Dowex 50WX8 (forme H+) avec une solution de 2M d'ammoniaque. Les fractions positives ninhydrines ont été collectées et le solvant enlevé. Le résidu a été recristallisé avec de l'H₂O/éthanol pour donner la (2S,3R,4S)-4-hydroxyisoleucine (1) (142 mg, 42%).

### Hydrogénolyse catalytique de (2S)-N-Bn-1

On a traité une solution de **(2S)-N-Bn-1** (85 mg, 0,36 mmol) dans de l'H₂O (10 ml) avec du H₂ en présence de 10% de palladium sur du carbone (5 mg) pendant 6 h à température ambiante. Le catalyseur a été enlevé par filtration et le solvant évaporé. Le résidu a été recristallisé à partir de H₂O/MeOH pour donner (1) (50 mg, 93%) : point de fusion = 224°C; [α]_{D} = +31,5° (c 1, H₂O); RMN ¹H (250 MHz, D₂O) δ 3,88 (d, J = 4,4 Hz, 1H); 3,78 (m, 1H); 1,91 (m, 1H); 1,23 (d, J = 6,4 Hz, 3H); 0,95 (d, J = 7,0 Hz, 3H); RMN ¹³C (50 MHz, D₂O) δ 174,2; 70,4; 57,5; 41,9; 21,3; 12,7; SM (ES) m/z 148 [M + H]⁺.

### Le (2S,3S)-anti-2-méthyl-3-benzyloxybutanoate d'éthyle (12)

A une solution de (2S,3S)-anti-2-méthyl-3-hydroxybutanoate d'éthyle (3) (1,0 g, 6,85 mmol) et de 2,2,2-trichloroacetimidate de benzyle (4,32 g, 17,12 mmol) dans du cyclohexane (20 ml) et du dichlorométhane (10 ml), on a ajouté, goutte à goutte, de l'acide trifluorométhanesulfonique (0,09 ml). On a agité le mélange réactionnel à température ambiante pendant 24 heures. On a ajouté du NaHCO₃ saturé. On a extrait la couche organique avec du dichlorométhane. On a réuni les extraits organiques, on les a séchés et concentrés. On a filtré le solide résultant et on l'a lavé avec de l'heptane. On a concentré le filtrat sous vide et on a chromatographié l'huile résultante sur une colonne de gel de silice en éluant avec de l'heptane/éther = 50:1 puis avec de l'heptane/EtOAc = 20:1 afin d'obtenir le (2S,3S)-anti-2-méthyl-3-benzyloxybutanoate d'éthyle (12) (1,305 g, 81%).

### Le (2R3S)-2-méthyl-3-benzyloxybutanol (13)

A une suspension agitée de LiAlH₄ (360 mg, 9,50 mmol) dans de l'éther (48 ml), on a ajouté, goutte à goutte, une solution de (2S,3S)-anti-2-méthyl-3-benzyloxy-butanoate d'éthyle (1,12 g, 4,75 mmol) à 0°C. On a agité le mélange réactionnel à 0°C pendant 30 min, puis à température ambiante pendant 4 heures. On a neutralisé la réaction au moyen du "traitement de Fieser": on a traité la réaction en ajoutant successivement, goutte à goutte, 0,36 ml d'eau glacée, 0,36 ml d'une solution aqueuse de NaOH à 15 % et 3 x 0,36 ml d'eau glacée. Après agitation pendant au moins 30 min, on a filtré le mélange à travers de la Célite. Ensuite on a lavé les sels d'aluminium précipités avec de l'EtOAc. On a évaporé le solvant, on a purifié l'huile résiduelle à travers du gel de silice (heptane/EtOAc = 5:1 puis 2:1) afin d'obtenir le (2R,3S)-2-méthyl-3-benzyloxybutanol (13) sous forme d'une huile incolore (673 mg, 73%): [α]_{D} = +65° (c 3,0 ; CHCl₃); IR (CHCl₃) 3501, 3011, 2978, 2934, 2880, 1497, 1455, 1423, 1376, 1350, 1236 cm⁻¹; RMN ¹H (250 MHz, CDCl₃) δ 7,39 à 7,25 (m, 5H); 4,66 (d, J = 11,5 Hz, 1H); 4,46 (d, J = 11,5 Hz ,1H); 3,65 (dd, J = 10,9, 3,9 Hz, 1H); 3,57 (dd, J = 10,9, 6,7 Hz, 1H); 3,49 (dq, J = 6,8, 6,2 Hz, 1H); 2,85 (s large, 1H, OH); 1,79 (sextuplet-d, J = 7,0, 3,9 Hz, 1H); 1,25 (d, J = 6,2 Hz, 3H); 0,90 (d, J = 7,0 Hz, 3H); RMN ¹³C (62,5 MHz, CDCl₃) δ 138,3; 128,2; 127,5; 127,4; 79,0; 70,5; 66,0; 40,7; 16,7; 13,3; SM (CI) m/z 195 [M + H]⁺. SMHR : Calculé pour C₁₂H₁₉O₂ (M + H), 195,13849; trouvé, 195,13830.

### Le (2S,3S)-2-méthyl-3-benzyloxybutyraldéhyde (14)

A une solution de (2R,3S)-2-méthyl-3-benzyloarybutanol **(13)** (830 mg, 4,28 mmol) et d'Et₃N (2,86g, 3,93 ml, 28,25 mmol, 6,6 éq.) dans du DMSO (14 ml), on a ajouté en portions, le complexe Py.SO₃ (4,09 g, 25,67 mmol, 6,0 éq.) à 0°C. On a agité le mélange réactionnel à 0°C pendant 3,5 heures puis à température ambiante pendant 2 heures, puis on l'a divisé entre de l'eau et de l'éther. On a extrait la couche aqueuse avec de l'éther. On a lavé les extraits éthérés réunis avec du HCl 1M, de l'eau, du NaHCO₃ saturé, de la saumure, on les a séchés sur Na₂SO₄ et on l'a évaporé pour obtenir le (2S,3S)-2-méthyl-3-benzyloxybutyraldéhyde brut que l'on a utilisé directement pour la réaction suivante sans purification. RMN ¹H (250 MHz, CDCl₃) δ 9,73 (d, J = 2,4 Hz, 1H); 4,63 (d, J = 11,7 Hz, 1H); 4,44 (d, J = 11,7 Hz, 1H); 3,81 (quintuplet, J = 6,4 Hz, 1H); 2,57 (quintuplet-d, J = 7,0; 2,4 Hz, 1H); 1,25 (d, J = 6,2 Hz, 3H); 1,09 (d, J = 7,1 Hz, 3H).

### La (2R,3S)-1-cyano-2-méthyl-3-benzyloxy-N-benzylbutylamine (15)

A une solution de l'aldéhyde préparé ci-dessus dans du dichlorométhane (21 ml) en présence de MgSO₄, on a ajouté de la benzylamine (550 mg, 561 µl, 5,14 mmol) à température ambiante et on a poursuivi l'agitation pendant une nuit à température ambiante. On a filtré le mélange réactionnel à travers de la Célite et on a évaporé le filtrat pour obtenir l'imine brute. A une solution de l'imine dans du dichlorométhane (15 ml) et du méthanol (5 ml) à 0°C, on a ajouté TMSCN (637 mg, 856 µl, 6,42 mmol). Après agitation à 0°C pendant 1 heure puis à température ambiante pendant une nuit, on a séparé le mélange réactionnel dans du NaHCO₃ saturé et de l'éther. On a extrait la phase aqueuse avec de l'éther, on a lavé les extraits éthérés avec de la saumure, on les a séchés et filtrés à travers un disque court de Célite et Na₂SO₄ et on les a évaporés. Une chromatographie éclair sur colonne de gel de silice avec pour éluant de l'heptane/EtOAc = 10:1, a produit la (1R,2R,3S)-1-cyano-2-méthyl-3-benzyloxy-N-benzylbutylamine **(1R)-(15)** (705 mg, 53%) et (1S,2R,3S)-1-cyano-2-méthyl-3-benzyloxy-N-benzylbutylamine **(1S)-(15)** (400 mg, 30%) [(1R)/(1S) = 1,76:1].

*La (1R,2R,3S)-1-cyano-2-méthyl-3-benzyloxy-N-benzylbutylamine **(1R)-(15)*** [α]_{D}=+114° (c 4,4; CHCl₃); IR (CHCl₃) 3338, 3089, 3067, 3030, 3013, 2979, 2936, 2905, 2882, 2226, 1605, 1587, 1497, 1455, 1386, 1377, 1363, 1340, 1231, 1222, 1216 cm⁻¹; RMN ¹H (250 MHz, CDCl₃) δ 7,32 à 7,18 (m, 10H); 4,58 (d, J = 10,6 Hz, 1H); 4,34 (d, J = 10,6 Hz, 1H); 3,95 (d, J = 12,7 Hz, 1H); 3,63 (d, J = 12,7 Hz, 1H); 3,74 à 3,62 (m, 2H); 2,22 (s large, 1 H, NH); 2,07 à 1,94 (m, 1H); 1,24 (d, J = 6,1 Hz, 3 H); 1,05 (d, J = 7,1 Hz, 3H); RMN ¹³C (62,5 MHz, CDCl₃) δ 138,4; 137,9; 128,2; 127,9; 127,5; 127,1; 119,6; 77,1; 71,0; 53,4; 51,7; 42,7; 17,0; 13,7; SM (CI) m/z 309 [M + H]⁺. SMHR : Calculé pour C₂₀H₂₅N₂O (M + H), 309,19668; trouvé, 309,19396.

*La (1S,2R,3S)-1-cyano-2-méthyl-3-benzyloxy-N-benzylbutylamine* **(*1S*)*-*(*15*)** [α]_{D}= -12° (c 3,6; CHCl₃); IR (CHCl₃) 3343, 3090, 3068, 3031, 3013, 2979, 2935, 2880, 2226, 1605, 1587, 1497, 1455, 1386, 1378, 1359, 1333, 1232 cm⁻¹; RMN ¹H (250 MHz, CDCl₃) δ 7,34 à 7,22 (m, 10H); 4,59 (d, J = 11,2 Hz, 1H); 4,37 (d, J = 11,2 Hz, 1H); 4,04 (d, J = 12,8 Hz, 1H); 3,97 (d, J = 4,7 Hz, 1H); 3,79 (d, J = 12,8 Hz, 1H); 3,50 (dq, J = 8,8; 6,1 Hz, 1H); 2,07 à 1,93 (m, 1 H); 1,62 (s large, 1 H, NH); 1,22 (d, J = 6,0 Hz, 3H); 1,04 (d, J = 6,9 Hz, 3H); RMN ¹³C (62,5 MHz, CDCl₃) δ 138,3; 138,0; 128,4; 128,3; 127,6; 127,5; 127,4; 119,1; 76,0; 70,8; 51,9; 51,5; 42,4; 16,4; 11,7; SM (CI) m/z 309 [M + H]⁺. SMHR : Calculé pour C₂₀H₂₅N₂O (M + H), 309,19668; trouvé, 309,19321.

### Le (3R,4R,5S)-3-N-benzylamino-4-méthyl-5-méthyl-2-oxo-tétrahydrofuranne (3R)-(11)

On a mis à reflux une solution de (1R,2R,3S)-1-cyano-2-méthyl-3-benzyloxy-N-benzylbutylamine **(1R)-(15)** (220 mg, 0,71 mmol) dans du HCl 6N (7 ml) pendant 6 heures. On a lavé le mélange réactionnel avec du dichlorométhane, on l'a basifié avec du NaHCO₃ aqueux saturé, on l'a extrait avec de l'EtOAc. On a lavé les extraits d'EtOAc avec de la saumure, on les a séchés et évaporés. Une CCM préparative sur du gel de silice (heptane/Et₂O = 1:1) a produit du (3R,4R,5S)-3-N-benzylamino-4-méthyl-5-méthyl-2-oxo-tétrahydrofuranne sous forme d'une huile incolore (124 mg, 80%): [α]_{D}=+48° (c 2,4; CHCl₃); IR (CHCl₃) 3528, 3327, 3030, 2980, 2934, 2913, 2877, 1770, 1604, 1455, 1389, 1328, 1231, 1224, 1211, 1187 cm⁻¹; RMN ¹H (250 MHz, CDCl₃) δ 7,39 à 7,23 (m, 5H); 4,06 à 3,94 (m, 3H); 3,18 (d, J = 11,4 Hz, 1H); 2,01 à 1,85 (m, 2H); 1,39 (d, J = 6,1 Hz, 3H); 1,14 (d, J = 6,5 Hz, 3H); RMN ¹³C (62,5 MHz, CDCl₃) δ 177,0; 139,6; 128,3; 128,0; 127,0; 79,5; 63,4; 51,3; 45,4; 18,4; 14,3; SM (CI) m/z (sel d'HCl) 220 [M + H]⁺. Le sel d'HCl: RMN ¹H (250 MHz, D₂O) δ 7,50 (m, 5H); 4,56 (d, J = 12,9 Hz, 1H); 4,45 à 4,34 (m, 1H); 4,39 (d, J = 12,9 Hz, 1H); 4,26 (d, J = 11,5 Hz, 1H); 2,58 à 2,41 (m, 1H); 1,46 (d, J = 6,2 Hz, 3H); 1,25 (d, J = 6,5 Hz, 3H); RMN ¹³C (62,5 MHz, D₂O) δ 173,0; 130,6; 130,4; 129,9; 82,6; 67,0; 61,4; 50,3; 42,0; 17,9; 13,6. SMHR : Calculé pour C₁₃ H₁₈NO₂ (M + H), 220,13374; trouvé, 220,13195.

### Le (3S,4R,5S)-3-N-benzylamino-4-méthyl-5-méthyl-2-oxo-tétrahydrofuranne (3S)-(11)

On a mis à reflux une solution de (1S,2R,3S)-1-cyano-2-méthyl-3-benzyloxy-N-benzylbutylamine **(1S)-(15)** (300 mg, 0,97 mmol) dans du HCl 6N (10 ml) pendant 6 heures. On a lavé le mélange réactionnel avec du dichlorométhane, on l'a basifié avec du NaHCO₃ aqueux saturé, on l'a extrait avec de l'EtOAc. On a lavé les extraits d'EtOAc avec de la saumure, on les a séchés et évaporés. Une CCM préparative sur gel de silice (dichlorométhane/acétone = 25:1) a produit du (3S,4R,5S)-3-N-benzylamino-4-méthyl-5-méthyl-2-oxo-tétrahydrofuranne sous forme d'une huile incolore (117 mg, 55%) : [α]_{D}=-14° (c 2,2; CHCl₃); IR (CHCl₃) 3528, 3333, 3088, 3066, 3029, 2981, 2935, 2875, 2822, 1770, 1605, 1496, 1455, 1384, 1361, 1300, 1229, 1178 cm⁻¹; RMN ¹H (250 MHz, CDCl₃) 7,38 à 7,18 (m, 5H); 4,31 (qd, J = 6,5, 3,5 Hz, 1H); 3,93 (d, J = 13,2 Hz, 1H); 3,86 (d, J = 13,2 Hz, 1H); 3,61 (d, J = 7,3 Hz, 1H); 2,26 (quintuplet-d, J = 7,1, 3,4 Hz, 1H); 1,75 (s large, 1 H, NH); 1,36 (d, J = 6,5 Hz, 3H); 1,07 (d, J = 7,1 Hz, 3H); RMN¹³C (62,5 MHz, CDCl₃) δ 176,5; 139,3; 128,4; 128,1; 127,2; 81,5; 58,1; 52,1; 40,1; 19,6; 12,6; SM (CI) m/z (sel d'HCl) 220 [M + H]⁺. Le sel d'HCl: RMN ¹H (250 MHz, D₂O) δ 7,52 (m, 5H); 4,70 (d, J = 7,7 Hz, 1H); 4,64 (m, 1H); 4,60 (d, J = 13,1 Hz, 1H); 4,42 (d, J = 13,1 Hz); 2,84 (quintuplet, J = 7,2 Hz, 1H); 1,42 (d, J = 6,7 Hz, 3H); 1,18 (d, J = 7,2 Hz, 3H); RMN¹³C ( 62,5 MHz, D₂O+CD₃OD) δ 173,1; 131,2; 131,0; 130,3; 84,9; 58,1; 52,0; 38,3; 19,5; 13,8. SMHR : Calculé pour C₁₃ H₁₈NO₂ (M + H), 220,13374; trouvé, 220,13562.

### Le (3R,4R,5S)-3-amino-4-méthyl-5-méthyl-2-oxo-tétrahydrofuranne (3R)-(16)

On a hydrogéné à température ambiante sous pression atmosphérique pendant une nuit, une suspension de chlorhydrate de (3R,4R,5S)-3-N-benzylamino-4-méthyl-5-méthyl-2-oxo-tétrahydrofuranne (120 mg, 0,47 mmol) et 10% de Pd-C (24 mg) dans du méthanol (10 ml). On a retiré le catalyseur par filtration, on a évaporé le solvant pour obtenir le (3R,4R,5S)-3-amino-4-méthyl-5-méthyl-2-oxo-tétrahydrofuranne (78 mg, 100%): [a]_{D}= -3,4° (c 1,6, MeOH); IR (nujol) 3411, 2923, 2853, 1783, 1762, 1588, 1557, 1491, 1457, 1390, 1340, 1300, 1207 cm⁻¹; RMN ¹H (300 MHz, D₂O) δ 4,47 (dq, J = 9,7; 6,2 Hz, 1H); 4,19 (d, J = 11,7 Hz, 1H); 2,46 à 2,32 (m, 1H); 1,49 (d, J = 6,2 Hz, 3H); 1,29 (d, J = 6,6 Hz, 3H); RMN ¹³C (62,5 MHz, D₂O) δ 174,1; 83,1; 56,5; 43,5; 18,1; 13,2; SM (CI) m/z 130 [M + H]⁺. SMHR : Calculé pour C₆H₁₂NO₂ (M + H), 130,08680; trouvé, 130,08625.

### Le (3S,4R,5S)-3-amino-4-méthyl-5-méthyl-2-oxo-tétrahydrofuranne (3S)-(16)

On a hydrogéné à température ambiante, sous pression atmosphérique, pendant une nuit, une suspension de chlorhydrate de (3S,4R,5S)-3-N-benzylamino-4-méthyl-5-méthyl-2-oxo-tétrahydrofuranne (110 mg, 0,43 mmol) et 10% de Pd-C (22 mg) dans du méthanol (9 ml). On a retiré le catalyseur par filtration. On a évaporé le solvant pour obtenir le (3S,4R,5S)-3-amino-4-méthyl-5-méthyl-2-oxo-tétrahydrofuranne (71 mg, 100%): [α]_{D}= -18° (c 1,4, MeOH); IR (nujol) 3526, 2924, 2854, 1771, 1581, 1504, 1462, 1385, 1355, 1315, 1260, 1209 cm⁻¹; RMN ¹H (250 MHz, D₂O) δ 4,61 à 4,54 (m, 2H); 2,72 (quintuplet large, J = 7,5 Hz, 1H); 1,40 (d, J = 6,6 Hz, 3H); 1,11 (d, J = 7,3 Hz, 3H); RMN ¹³C (50 MHz, D₂O) δ 174,3; 85,2; 52,3; 38,1; 19,5; 13,0; SM (CI) m/z 130 [M + H]⁺. SMHR ; Calculé pour C₆H₁₂NO₂ (M + H), 130,08680; trouvé, 130,08604.

### Le (3R,4R,5S)-3-N-benzylamino-4-méthyl-5-méthyl-2-oxo-tétrahydrofuranne (3R)-(11) à partir de la (1R,2R,3S)-1-cyano-2-méthyl-3-tétrahydropyranyloxy-N-benzyl-butylamine (1R)-(10)

On a mis à reflux pendant 6 heures une solution de (1R,2R,3S)-1-cyano-2-méthyl-3-tétrahydropyranyloxy-N-benzylbutylamine **(1R)-(10)** (25 mg, 0,083 mmol) dans du HCl 6N (2 ml). On a lavé le mélange réactionnel avec du dichlorométhane et on l'a évaporé afin d'obtenir le chlorhydrate de (3R,4R,5S)-3-benzylamino-4-méthyl-5-méthyl-2-oxo-tétrahydrofuranne **(3R)-(11)** (21 mg, 100%).

### Le (3S,4R,5S)-3-N--benzylamino-4-méthyl-5-méthyl-2-oxo-tétrahydrofuranne (3S)-(11) à partir de la (1S,2R,3S)-1-cyano-2-méthyl-3-tétrahydropyranyloxy-N-benzyl-butylamine (1S)-(10)

On a mis à reflux pendant 6 heures une solution de (1S,2R,3S)-1-cyano-2-méthyl-3-tétrahydropyranyloxy-N-benzylbutylamine (34 mg, 0,11 mmol) dans du HCl 6N (2 ml). On a lavé le mélange réactionnel avec du dichlorométhane, on l'a basifié avec du NaHCO₃ aqueux saturé, on l'a extrait avec de l'EtOAc. On a lavé les extraits d'EtOAc avec de la saumure, on les a séchés et évaporés pour obtenir le (3S,4R,5S)-3-N-benzylamino-4-méthyl-5-méthyl-2-oxo-tétrahydrofuranne (une γ-lactone) (24 mg, 100%).

### La (2R,3S)-1-cyano-2-méthyl-3-tétrahydropyranyloxy-N-[(S)-1'-phényléthyl]-butylamine (17)

A une solution de (2S,3S)-2-méthyl-3-tétrahydropyranyloxybutyraldéhyde **(6)** (270 mg, 1,45 mmol) dans du dichlorométhane (6 ml), on a ajouté de la (S)-(-)-1-phényléthylamine (211 mg, 225 µl, 1,74 mmol) à température ambiante, et on a poursuivi l'agitation à température ambiante pendant 2 heures. On a refroidi le mélange réactionnel à 0°C, on a introduit successivement du méthanol (2 ml) et du TMSCN (216 mg, 290 µl, 2,18 mmol). Après agitation à 0°C pendant 2 heures puis à température ambiante pendant 22 heures, on a séparé le mélange réactionnel dans du NaHCO₃ saturé et de l'éther. On a extrait la phase aqueuse avec de l'éther, on a lavé les extraits d'éther avec de la saumure, on les a séchés et filtrés à travers un disque court de Célite et de Na₂SO₄ puis évaporés. Une chromatographie éclair sur une colonne de gel de silice avec pour éluant un mélange heptane/EtOAc =10:1 puis 8:1, a produit la (1R,2R,3S)-1-cyano-2-méthyl-3-tétrahydropyranyloxy-N-[(S)-l'-phényléthyl]butylamine **(1R)-(17)** et la (1S,2R,3S)-1-cyano-2-méthyl-3-tétra-hydropyranyloxy-N-[(S)-1'-phényléthyl]butylamine **(1S)-(17)** (au total 414 mg, 90%) dans le rapport d'environ 1:3.

### La (1R,2R,3S)-1-cyano-2-méthyl-3-tétrahydropyranyloxy-N-[(S)-1'-phényléthyl]-butylamine (1R)-17 et la (1S,2R,3S)-1-cyano-2-méthyl-3-tétrahydropyranyloxy-N-[(S)-1'-phényléthyl]butylamine (1S)-17

### Premier procédé :

A une suspension de (2S,3S)-2-méthyl-3-tétrahydropyranyloxybutyraldéhyde **(6)** (1,23 g, 6,61 mmol) dans du méthanol (33ml) et de l'eau (33ml), on a ajouté du chlorhydrate de (S)-(-)-phényléthylamine (1,038 g, 6,61 mmol) et du KCN (432 mg, 6,61 mmol) à température ambiante. Après la poursuite de l'agitation à température ambiante pendant 24 heures, on a séparé le mélange réactionnel dans du NaHCO₃ saturé et de l'acétate d'éthyle. On a extrait la phase aqueuse avec de l'acétate d'éthyle, on a lavé les extraits organiques avec de la saumure, on les a séchés et filtrés à travers un disque court de Célite et du Na₂S0₄ puis évaporés. Une chromatographie éclair sur une colonne de gel de silice avec pour éluant un mélange heptane/EtOAc = 10:1 puis 8:1, a produit le mélange de la (1R,2R,3S)-1-cyano-2-méthyl-3-tétrahydropyranyloxy-N-[(S)-1'-phényléthyl]butylamine **(1R)-(17)** et la (1S,2R,3S)-1-cyano-2-méthyl-3-tétrahydropyranyloxy-N-[(S)-1'-phényléthyl]butylamine **(1S)-(17)** (1,73 g, 83%).

### Deuxième procédé :

A une suspension de (2S,3S)-2-méthyl-3-tétrahydropyranyloxybutyraldéhyde (6) (1,23 g, 6,61 mmol) dans du méthanol (33ml) et de l'eau (33ml), on a ajouté du chlorhydrate de (S)-(-)-phényléthylamine (1,038 g, 6,61 mmol) et du KCN (432 mg, 6,61 mmol) à 0°C. Après poursuite de l'agitation à 0°C pendant 30 minutes puis à température ambiante pendant 48 heures, on a séparé le mélange réactionnel dans de l'eau et de l'acétate d'éthyle. On a extrait la phase aqueuse avec de l'acétate d'éthyle, on a lavé les extraits organiques avec de la saumure, on les a séchés et filtrés à travers un disque court de Célite et du Na₂SO₄ puis évaporés. Une chromatographie éclair sur une colonne de gel de silice avec pour éluant un mélange heptane/EtOAc = 10:1 puis 8:1, a produit le mélange de la (1R,2R,3S)-1-cyano-2-méthyl-3-tétrahydropyranyloxy-N-[(S)-1'-phényléthyl]butylamine (1R)-(17) et la (1S,2R,3S)-1-cyano-2-méthyl-3-tétrahydropyranyloxy-N-[(S)-1'-phényléthyl]butylamine (1S)-(17) (1,73 g, 83%).

*La* (*1S,2R,3S*)*-1-cyano-2-méthyl 3-tétrahydropyranyloxy-N-[(S)-1'-phényléthyl]-butylamine **(1S)-(17)***
IR (CHCl₃) 3500, 3316, 3028, 3012, 2968, 2947, 2854, 2226, 1494, 1453, 1376, 1356, 1275, 1260, 1234, 1186, 1132 cm⁻¹; RMN ¹H (250 MHz, CDCl₃) δ 7,39 à 7,23 (m, 5H); 4,59 (m, 0,5H); 4,50 à 4,47 (m, 0,5H); 4,11 à 4,03 (m, 1H); 3,88 à 3,74 (m, 1H); 3,68 à 3,55 (m, 1H); 3,46 à 3,40 (m, 2H); 1,92 à 1,34 (m, 7H); 1,40 (d, J = 6,5 Hz, 1,5H); 1,38 (d, J = 6,4 Hz, 1,5H); 1,15 (d, J = 6,2 Hz, 1,5H); 1,06 (d, J = 6,1 Hz, 1,5H); 1,02 (d, J = 6,9 Hz, 1,5H); 1,00 (d, J = 6,9 Hz, 1,5H); RMN ¹³C (62,5 MHz, CDCl₃) δ 143,6; 143,1; 128,6; 128,5; 127,5; 127,4; 127,0; 126,9; 120,1; 119,5; 100,2; 96,0; 76,4; 71,4; 63,2; 62,7; 56,6; 56,4; 50,5; 50,4; 42,7; 31,0; 30,7; 25,3; 24,7; 24,6; 19,9; 19,7; 18,5; 16,4; 13,1; 11,7; SM (CI) m/z 317 [M + H]⁺. SMHR : Calculé pour C₁₉H₂₉N₂O₂ (M + H), 317,22289; trouvé, 317,22647.

### Le (35,4R,5S)-3-N-[(S)-1'-phényléthylamino]-4-méthyl-5-méthyl-2-oxo-tétrahydrofuranne (3S)-(18) et le (3R,4R,5S)-3-N-[(S)-1'-phényléthylamino]-4-méth-5-méthyl-2-oxo-tétrahydrofuranne (3R)-(18)

On a mis à reflux pendant 6 heures une solution de (2R,3S)-1-cyano-2-méthyl-3-pyranyloxy-N-[(S)-1'-phényléthyl]butylamine **(17)** (180 mg, 0,57 mmol) dans du HCl 6N (11 ml). On a lavé le mélange réactionnel avec de l'EtOAc / heptane. A partir de là, deux procédés ont permis d'obtenir soit un mélange des composés (3S)-18 et (3R)-18, soit le composé (3S)-18 seul.

Le premier procédé est le suivant : on a basifié avec du NaHCO₃ aqueux saturé le mélange réactionnel lavé et on l'a extrait avec de l'EtOAc. On a lavé les extraits d'EtOAc avec de la saumure, on les a séchés et évaporés. Une chromatographie éclair sur une colonne de gel de silice (heptane/EtOAc = 6:1) a produit le (3S,4R,5S)-3-N-[(S)-1'-phényléthylamino]-4-méthyl-5-méthyl-2-oxo-tétrahydrofuranne **(3S)-(18)** (57 mg, 43%) et le (3R,4R,5S)-3-N-[(S)-1'-phényl-éthylamino]-4-méthyl-5-méthyl-2-oxo-tétrahydrofuranne **(3R)-(18)** (16 mg, 12%) [(3S)/(3R) = 3,5:1].

Le deuxième procédé est le suivant :

L'eau contenu dans le mélange réactionnel lavé a été éliminée par évaporation totale . Une cristallisation est ensuite réalisée avec de l'isopropanol de façon à obtenir le (3 S,4R,5S)-3-N-[(S)-1'-phényléthylamino]-4-méthyl-5-méthyl-2-oxo-tétrahydrofuranne (3S)-(18) avec un rendement à partir du composé (6) de 53%.

*Le (3R, 4R,5S)-3-N-[(S)-1*'*phényléthylamino]-4-méthyl-5-méthyl 2-oxo-tétrahydrofuranne **(3R)-(18)***
[α]_{D}= -39° (c 0,7, CHCl₃); IR (CHCl₃) 3693, 3329, 3030, 2967, 2933, 2877, 1765, 1603, 1494, 1453, 1388, 1329, 1247, 1175 cm⁻¹; RMN ¹H (250 MHz, CDCl₃) δ 7,38 à 7,24 (m, 5H); 4,01 (q, J = 6,6 Hz, 1H); 3,89 (qd, J = 6,1, 9,7 Hz, 1H); 3,08 (d, J = 11,1 Hz, 1H); 2,05 (s large, 1H, NH); 1,91 (m, 1H); 1,38 (d, J = 6,6 Hz, 3H); 1,35 (d, J = 6,1 Hz, 3H); 1,12 (d, J = 6,5 Hz, 3H); RMN ¹³C (62,5 MHz, CDCl₃) δ 177,2; 144,7; 128,7; 127,2; 126,4; 79,5; 61,9; 56,5; 46,9; 24,7; 18,4; 14,8; SM (CI) m/z 234 [M + H]⁺. SMHR : Calculé pour C₁₄H₂₀NO₂ (M + H), 234,14939; trouvé, 234,15006.

*Le (3S, 4R, 5S)-3-N-[(S)-1*'*-phényléthylamino]-4-méthyl-5-méthyl-2-oxo-tétrahydrofuranne **(3S)-(18)***
[α]_{D}= -94° (c 1,7, CHCl₃); IR (CHCl₃) 3568, 3330, 3028, 2980, 2933, 2875, 1769, 1494, 1453, 1383, 1354, 1301, 1224, 1220, 1172, 1146 cm⁻¹; RMN ¹H (250 MHz, CDCl₃) δ 7,39 à 7,22 (m, 5H); 4,23 (qd, J = 6,5, 3,7 Hz, 1H); 4,17 (q, J = 6,6 Hz, 1H); 3,38 (d, J = 7,4 Hz, 1H); 1,89 (quintuplet-d, J = 7,2, 3,7 Hz, 1H); 1,61 (s large, 1H, NH); 1,38 (d, J = 6,6 Hz, 3H); 1,27 (d, J = 6,5 Hz, 3H); 0,99 (d, J = 7,1 Hz, 3H); RMN ¹³C (62,5 MHz, CDCl₃) δ 117,5; 144,8; 128,5; 127,3; 127,1; 81,3; 57,4; 57,0; 40,6; 24,6; 19,6; 12,7; SM (CI) m/z 234 [M + H]⁺. Le sel d'HCl: RMN ¹H (250 MHz, D₂O) δ 7,54 (s, 5H); 4,88 (q, J = 6,9 Hz, 1H); 4,53 (q, J = 6,6 Hz, 1H); 4,27 (d, J = 7,7 Hz, 1H); 2,60 (quintuplet, J = 7,3 Hz, 1H); 1,72 (d, J = 6,9 Hz, 3H); 1,26 (d, J = 6,7 Hz, 3H); 1,12 (d, J = 7,2 Hz, 3H); RMN ¹³C (62,5 MHz, CD₃OD) δ 171,6; 136,6; 130,9; 130,5; 129,3; 83,9; 59,3; 56,2; 39,0; 20,0; 19,8; 14,5. SMHR : Calculé pour C₁₄H₂₀NO₂ (M + H), 234,14939; trouvé, 234,15075.
Point de fusion : 228-229°C.

### Le (3S,4R,5S)-3-amino-4-méthyl-5-méthyl-2-oxo-tétrahydrofuranne (3S)-(16) à partir du (3S,4R,5S)-3-N-[(S)-1'-phényléthylamino]-4-méthyl-5-méthyl-2-oxo-tétrahydrofuranne (3S)-(18). HCl

On a hydrogéné à température ambiante, sous pression atmosphérique, pendant une nuit une suspension de chlorhydrate de (3S,4R,5S)-3-N-[(S)-1'-phényléthylamino]-4-méthyl-5-méthyl-2-oxo-tétrahydrofuranne (1,20 g, 4,46 mmol) et 10% de Pd-C (237 mg) dans du méthanol (45 ml). On a retiré le catalyseur par filtration. On a évaporé le solvant pour obtenir le chlorhydrate de (3S,4R,5S)-3-amino-4-méthyl-5-méthyl-2-oxo-tétrahydrofuranne (3S)-(16) (734 mg, 100 %).

### La (2R,3S)-1-cyano-2-méthyl-3-tétrahydropyranyloxy-N-[(R)-1'-phényl-2'-hydroxy -éthyl]butylamine (19)

A une solution de (2S,3S)-2-méthyl-3-tétrahydropyranyloxybutyraldéhyde (6) (130 mg, 0,7 mmol) dans du dichlorométhane (6 ml), on a ajouté du (R)-phénylglycinol (115 mg, 0,84 mmol) à température ambiante et on a poursuivi l'agitation pendant 2 heures à température ambiante. On a refroidi le mélange réactionnel à 0°C, on a introduit successivement du méthanol (2 ml) et du TMSCN (104 mg, 140 µl, 1,05 mmol). Après agitation à 0°C pendant 2 heures puis à température ambiante pendant 22 heures, on a séparé le mélange réactionnel dans du NaHCO₃ saturé et dans de l'éther. On a extrait la phase aqueuse avec de l'éther, on a lavé les extraits éthérés avec de la saumure, on les a séchés et filtrés à travers un disque court de Célite et de Na₂SO₄ puis évaporés. Une chromatographie éclair sur une colonne de gel de silice avec pour éluant un mélange heptane/EtOAc = 3:1 puis 2:1, a produit un mélange de (1R,2R,3S)-1-cyano-2-méthyl-3-tétrahydropyranyloxy-N-[(R)-1'-phényl-2'-hydroxyéthyl]butylamine **(1R)-(19)** et de (1S,2R, 3S)-1-cyano-2-méthyl-3-tétrahydropyranyloxy-N-[(R)-1'-phényl-2'-hydroxyéthyl]butylamine **(1S)-(19)** (au total 225 mg, 97%) : IR (CHCl₃) 3630, 3442, 3347, 3012, 2947, 2856, 1493, 1455, 1385, 1356, 1231, 1173 cm⁻¹; RMN ¹H (250 MHz, CDCl₃) δ 7,37 à 7,27 (m, 5H); 4,72 à 4,39 (m, 1H); 4,14 à 3,39 (m, 7H); 2,30 (s large, 2H, OH+NH); 2,05 à 1,20 (m, 7H); [1,17 (d, J = 6,3 Hz), 1,08 (d, J = 7,7 Hz), 1,05 (d, J = 7,0 Hz), 6H]; RMN ¹³C (62,5 MHz, CDCl₃) δ 140.6; 140,0; 138,7; 138,4; 128.6; 128,0; 127,8; 127,7; 127,3; 120,4; 119,8; 119.6; 119,2; 101,0; 100,1; 97,5; 95.6; 78,9; 76,3; 71,4; 71,2; 67,1; 66,9; 66,0; 65,6; 65,5; 63,2; 63,0; 62,8; 62,7; 53,0; 50,5; 50,1; 49,9; 42,7; 42,5; 42,3; 31,0; 30,8; 30.6; 25,2; 25,0; 21,6; 20,0; 19.6; 19,4; 18,3; 17,0; 16,1; 14,0; 12,7; 12,0; 11,7. SM (CI) m/z 333 [M + H]⁺. SMHR : Calculé pour C₁₉H₂₉N₂O₃ (M + H), 333,21780; trouvé, 333,21668.

### Le (3S,4R,5S)-3-N-[(R)-1'-phényl-2'-hydroxyéthylamino]-4-méthyl-5-méthyl-2-oxo-tétrahydrofuranne (3S)-(20) et le (3R,4R,5S)-3-N-[(R)-1'-phényl-2'-hydroxy-éthylamino]-4-méthyl-5-méthyl-2-oxo-tétrahydrofuranne (3R)-(20)

On a mis à reflux pendant 6 heures, une solution de (2R,3S)-1-cyano-2-méthyl-3-tétrahydropyranyloxy-N-[(R)-1'-phényl-2'-hydroxyéthyl]butylamine (168 mg, 0,51 mmol) dans du HCl 6N (10 ml). On a lavé le mélange réactionnel avec du dichlorométhane, on l'a basifié avec du NaHCO₃ aqueux saturé et on l'a extrait avec de l'EtOAc. On a lavé les extraits d'EtOAc avec de la saumure, on les a séchés et évaporés. Une chromatographie préparative sur couche mince de gel de silice (heptane/EtOAc = 1:3) a produit du (3S,4R,5S)-3-N-[(R)-1'-phényl-2'-hydroxy-éthylamino]-4-méthyl-5-méthyl-2-oxo-tétrahydrofuranne **(3S)-(20)** (62 mg, 49%) et du (3R,4R,5S)-3-N-[(R)-1'-phényl-2'-hydroxyéthylamino]-4-méthyl-5-méthyl-2-oxo-tétra-hydrofuranne **(3R)-(20)** (32 mg, 25%) [(3S)/(3R) = 2:1].

*Le* (*3S,4R,5S)-3-N-[(R)-1'-phényl-2'-hydroxyéthylamino]-4-méthyl-5-méthyl -2-oxo-tétrahydrofuranne **(3S)-(20)***
[α]_{D}= -112° (c 1,4, CHCl₃); IR (CHCl₃) 3596, 3463, 3029, 3014, 2981, 2935, 2877, 1767, 1654, 1493, 1455, 1384, 1356, 1225, 1216, 1176 cm⁻¹; RMN ¹H (250 MHz, CDCl₃) δ 7,40 à 7,27 (m, 5H); 4,33 à 4,24 (m, 2H); 3,78 (dd, J = 11,1, 4,1 Hz, 1H); 3,59 (dd, J= 11,1; 8,8 Hz, 1H); 3,44 (d, J = 7,4 Hz, 1H); 2,37 (s large, 2H, OH+NH); 2,10 à 1,97 (m, 1H); 1,31 (d, J = 6,4 Hz, 3H); 1,06 (d, J = 7,1 Hz, 3H); RMN ¹³C (50 MHz, CDCl₃) δ 177,8; 140,1; 128,5; 127,7; 81,4; 67,3; 63,3; 57,1; 40,7; 19,4; 12,3; SM (CI) m/z 250 [M + H]⁺. SMHR : Calculé pour C₁₄H₂₀NO₃ (M + H), 250,14431; trouvé, 250,14316.

### Résolution enzymatique du (3SR,4R,5S)-3-N-benzylamino-4-méthyl-5-méthyl-2-oxo-tétrahydrofuranne (3RS)-(11)

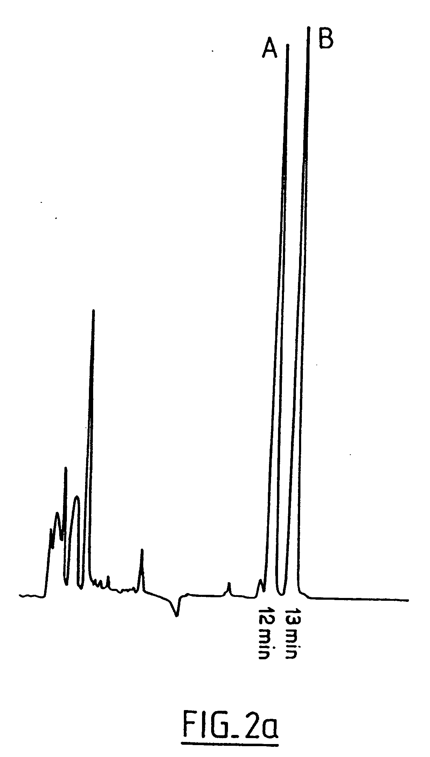

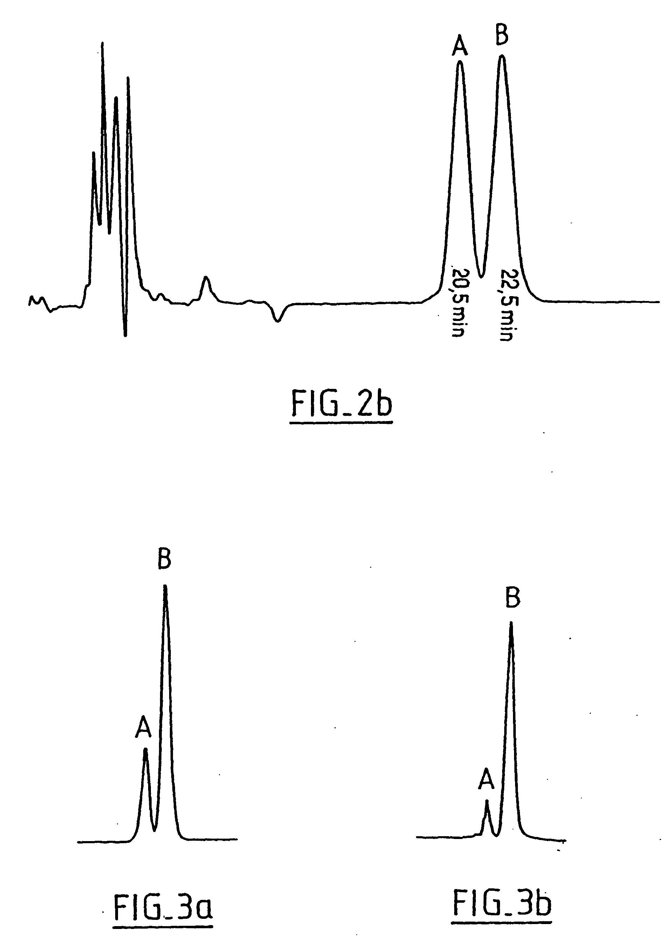

On a obtenu la (2R,3R,4S)-N-benzyl-4-hydroxyisoleucine au moyen d'une hydrolyse enzymatique diastéréosélective de la lactone **(11)**. Cette réaction a été catalysée soit par une préparation brute enzymatique ou à l'aide de cellules microbiennes vivantes. Dans une expérimentation typique, on a dissous la lactone **(11)** dans un tampon de phosphate (40 mM, pH 7,4) et on l'a agitée dans un agitateur rotatif à 27°C. On a analysé les échantillons par HPLC (figure 2, à 00 heures, A et B correspondent respectivement à **(3R)-(11)** et **(3S)-(11)).** Comme il est indiqué dans la figure 3, on a hydrolysé progressivement le **(3R)-(11),** tandis que le **(3S)-(11)** reste intact. On a extrait ce dernier avec de l'acétate d'éthyle et on l'a soumis à une autre hydrolyse enzymatique (non-stéréospécifique) afin d'obtenir la (2R,3R,4S)-N-benzyl-4-hydroxyisoleucine. Une hydrogénolyse catalytique de cette acide aminé N-protégé a donné (1).

### Synthèse de (1) à partir de (17)

### a) Synthèse du (3S,4R,5S)-3-N-[(S)-1'-phényléthylamino]-4-méthyl- 5-méthyl-2-oxo-tétrahydrofuranne (3S)-(18) et du (3R,4R,5S)-3-N- [(S)-1'-phényléthylamino]-4-méthyl-5-méthyl-2-oxo-tétrahydrofuranne (3R)-(18) à partir de (17)

Une solution d'un mélange de (1R,2R,3S) et de (1S,2R,3S)-1-cyano-2-méthyl-3-pyranyloxy-N-[(S)-1'-phényléthyl]butylamine (1,73 g, 5,47 mmol), **(1R)-17** et **(1S)-17,** respectivement, dans du HCl 6N (110 ml) a été mis à reflux pendant 6 heures. Le mélange réactionnel a été lavé 3 fois avec de l'EtOAc/heptane (1:1). La phase aqueuse a été évaporée pour donner le mélange de (3S,4R,5S)-3-N-[(S)-1'-phényléthylamino]-4-méthyl-5-méthyl-2-oxo-tétrahydrofuranne **(3S)-(18)** et le (3R,4R,5S)-3-N-[(S)-1'-phényléthylamino]-4-méthyl-5-méthyl-2-oxo-tétrahydrofuranne **(3R)-(18) ((2S)/(2R)** = 4,5 :1). Le résidu ainsi obtenu a été directement utilisé dans l'étape suivante sous forme de sel d'HCl.

### b) Synthèse de (1) à partir de (18)

Un mélange de (3S) et **(3R)-18** préparé ci-dessus a été dissout dans de l'eau (110 ml) puis traité avec du LiOH**·**H₂O (459 mg, 10,94 mmol) pendant de 24 heures à température ambiante. Du TFA (2,2 ml) a été ajouté au mélange réactionnel et le solvant a été évaporé immédiatement sous vide à 40-45°C. Le résidu a été dissout dans de l'eau et extrait avec de l'EtOAc. La phase aqueuse a été hydrogénée en présence de 10% de Pd-C pendant toute la nuit à température ambiante et sous pression atmosphérique. Le catalyseur a été enlevé par filtration. Le solvant a été évaporé et le résidu dissout dans de l'eau et passé à travers une colonne de résine échangeuse d'ions Dowex 50WX8 (forme H+). La colonne a été lavée minutieusement avec de l'eau et l'amino acide a été élué avec du NH₄OH 2M pour obtenir le composé (1) (423 mg, 59% pour trois étapes). Point de fusion = 224°C; [α]_{D}²⁰= +31,5° (c 1, H₂O); RMN ¹H (250 MHz, D₂O) δ 3,88 (d, J = 4,4 Hz, 1H); 3,78 (m, 1H); 1,91 (m, 1H); 1,23. (d, J = 6,4 Hz, 3H); 0,95 (d, J = 7 Hz, 3H); RMN ¹³C (50 MHz, D₂O) δ 174,2; 70,4; 57,5; 41,9; 21,3; 12,7; SM (ES) m/z 148 [M + H]⁺. Microanalyse : Calculé pour C₆H₁₃NO₃, C, 48,97; H, 8,90; N, 9,52; trouvé, C, 49,04; H, 8,83; N, 9,60.

*Le (2R)-N-(1 '-Phényléthyle)-*1
RMN ¹H (250 MHz, D₂O) δ 7,44 à 7,37 (m, 5H); 4,10 (q, J = 6,8 Hz, 1H); 3,85 (quintet, J = 6,2 Hz, 1H); 3,72 (d, J = 3,2 Hz, 1H); 1,95 (m, 1H); 1,52 (d, J = 6,8 Hz, 3H); 1,25 (d, J = 6,4 Hz, 3H); 0,91 (d, J = 7,3 Hz, 3H); RMN ¹³C (50 MHz, D₂O + CD₃OD) δ 172,9; 137,5; 130,6; 130,3; 128,7; 71,3; 63,4; 59,3; 40,7; 22,1; 18,5; 13,7.

*Le (25)-N-(1 '-Phényléthyle)-1*
Point de fusion = 155-157°C (décomposition); [α]_{D}= -25° (c 1,0, H₂O); RMN ¹H (250 MHz, D₂O) δ 7,42 à 7,32 (m, 5H); 4,23 (q, J = 6,8 Hz, 1H); 3,48 (m, 1H); 3,34 (d, J = 5,8 Hz, 1H); 1,64 (m, 1H); 1,60 (d, J = 6,8 Hz, 3H); 1,00 (d, J = 6,2 Hz, 3H); 0,76 (d, J = 6,9 Hz, 3H); RMN ¹³C (50 MHz, D₂O)δ 172,0; 135,1; 129,5; 129,2; 127,5; 70,6; 63,4; 58,3; 40,7; 20,5; 18,9; 11,9.

## Revendications

1. Procédé de synthèse de composés de formule générale (2S)-I suivante : 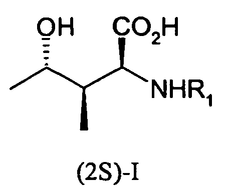 dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle
comportant l'étape a) de réduction diastéréo et énantiosélective du composé de formule 2 suivante : 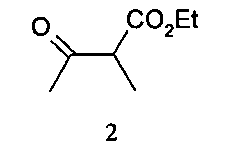 pour obtenir le composé de formule 3 suivante : 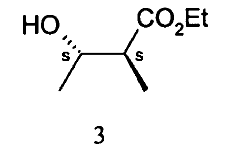 avec un excès énantiomérique d'au moins 85%, de préférence d'au moins 90%, et un excès diastéréomérique d'au moins 90 %, de préférence d'au moins 95%.

2. Procédé selon la revendication 1, **caractérisé en ce que** la réduction diastéréo et énantiosélective se fait par utilisation d'une enzyme.

3. Procédé selon la revendication 2, **caractérisé en ce que** l'on utilise un micro-organisme contenant cette enzyme, en particulier le *Geotrichum candidum.*

4. Procédé selon l'une quelconque des revendications précédentes, **caractérisé en ce qu'**il comporte les étapes supplémentaires de :
b) protection du groupe OH de l'alcool de formule 3 par un groupe Y,
c) réduction du groupe -COOEt en alcool et
d) oxydation du groupe OH non protégé obtenu en c) pour obtenir l'aldéhyde de formule générale **II** suivante : 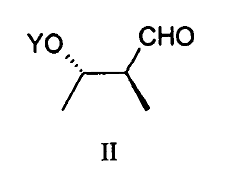
dans laquelle le groupe Y représente un groupe protecteur du groupe OH.

5. Procédé selon la revendication 4, **caractérisé en ce qu'**il comporte une étape supplémentaire e₁) de traitement du composé de formule générale II avec une sulfinamide de configuration (S) (+) pour obtenir le composé de formule générale IV suivante : 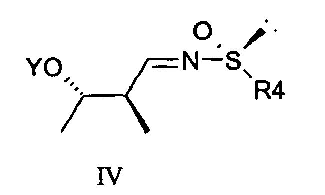 dans laquelle
le groupe Y représente un groupe protecteur du groupe OH
et le groupe R4 représente un groupe alkyle en C₁-C₆, aryle ou aralkyle.

6. Procédé selon la revendication 5, **caractérisé en ce qu'**il comporte une étape supplémentaire f₁) de traitement du composé de formule générale **IV** pour obtenir l'aminonitrile de formule générale V suivante : 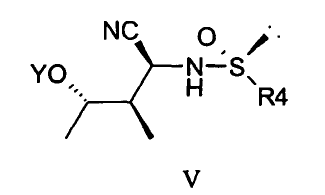 dans laquelle
le groupe Y représente un groupe protecteur du groupe OH
et le groupe R4 représente un groupe alkyle en C₁-C₆, aryle ou aralkyle.

7. Procédé selon la revendication 6, **caractérisé en ce qu'**il comporte une étape supplémentaire g₁) de traitement avec un acide de l'aminonitrile de formule générale V pour obtenir le sel de la lactone de formule 9 suivante :

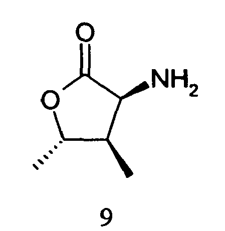

8. Procédé selon la revendication 7, **caractérisé en ce qu'**il comporte une étape supplémentaire h₁) d'hydrolyse alcaline du sel de la lactone de formule 9 pour obtenir le composé de formule générale (2S)-I dans lequel le groupe R₁ représente l'atome d'hydrogène.

9. Procédé selon la revendication 4, **caractérisé en ce qu'**il comporte une étape supplémentaire e₂) de traitement du composé de formule générale II pour obtenir l'aminonitrile de formule générale III suivante : 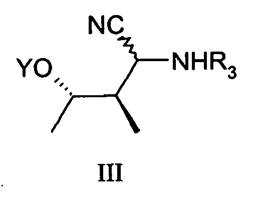 dans laquelle :
le groupe Y représente un groupe protecteur du groupe OH
et le groupe R₃ représente un groupe protecteur du groupe amine.

10. Procédé selon la revendication 9, **caractérisé en ce qu'**il comporte une étape supplémentaire f₂) de traitement avec un acide de l'aminonitrile de formule générale III pour obtenir la lactone de formule générale VI suivante : 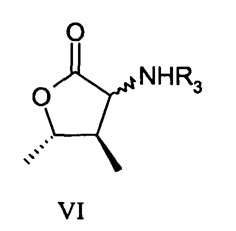 dans laquelle le groupe R₃ représente l'atome d'hydrogène ou un groupe protecteur du groupe amine
suivie si nécessaire par une étape supplémentaire f_{2,1}) de traitement de la lactone de formule générale VI pour obtenir la lactone de formule générale VII suivante : 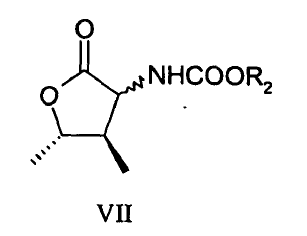 dans laquelle le groupe R₂ représente un groupe alkyle en C₁-C₆, un groupe aryle ou aralkyle.

11. Procédé selon la revendication 10, **caractérisé en ce qu'**il comporte une étape supplémentaire g₂) d'hydrolyse diastéréosélective de la lactone de formule générale VI ou VII pour obtenir le composé de formule générale (2S)-I.

12. Procédé selon la revendication 11 dans le cas où le groupe R₃ représente le groupe protecteur du groupe amine et le groupe R₁ représente un groupe protecteur du groupe amine ou un groupe de formule -COOR₂ dans laquelle le groupe R₂ représente un groupe alkyle en C₁-C₆, un groupe aryle ou aralkyle, **caractérisé en ce que** l'hydrolyse diastéréosélective consiste dans :
- L'hydrolyse alcaline de la lactone de formule générale VI dans laquelle le groupe R₃ représente un groupe protecteur du groupe amine ou de formule générale VII pour obtenir un mélange de diastéréomères de formule générale I suivante : 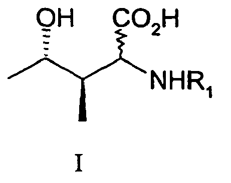
dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
- L'ajout d'acide organique, de préférence TFA, sous forme de traces, et le chauffage pour recycliser le composé de formule générale (2R)-I suivante : 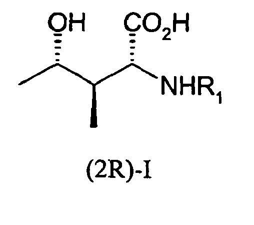
dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
en lactone de formule générale (3R)-VIII suivante : 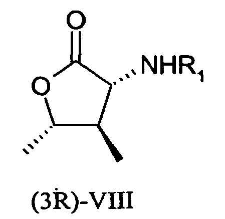 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
- L'extraction avec un solvant organique de la lactone de formule générale (3R)-VIII pour récupérer le composé de formule générale (2S)-I dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle.

13. Procédé selon la revendication 11, **caractérisé en ce que** l'hydrolyse diastéréosélective consiste dans :
- L'hydrolyse enzymatique diastéréosélective de la lactone de formule générale (3R)-VI ou (3R)-VII pour donner le composé de formule générale (2R)-I,
- L'extraction avec un solvant organique de la lactone non hydrolysée restante de formule générale (3S)-VIII suivante : 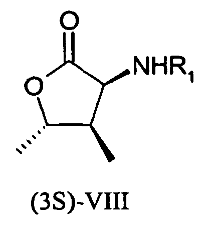
dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle,
- L'hydrolyse enzymatique non sélective de la lactone de formule générale (3S)-VIII pour obtenir le composé de formule générale (2S)-I dans laquelle le groupe R₁ représente l'atome d'hydrogène, un groupe protecteur du groupe amine
ou un groupe de formule -COOR₂ dans laquelle le groupe R₂ représente un groupe alkyle en C₁-C₆, un groupe aryle ou aralkyle.

14. Procédé selon la revendication 11 dans le cas où le groupe R₃ représente le groupe protecteur du groupe amine **caractérisé en ce que** l'hydrolyse diastéréosélective consiste dans :
- la cristallisation dans un alcool de la lactone de formule générale VI dans un solvant organique de façon à obtenir le sel du composé de formule (3S)-VIII dans laquelle le groupe R₁ représente un groupe protecteur du groupe amine,
- l'hydrogénolyse catalytique du sel du composé de formule (3S)-VIII dans laquelle le groupe R₁ représente un groupe protecteur du groupe amine de façon à obtenir le sel de la lactone de formule 9,
- l'hydrolyse alcaline du sel de la lactone de formule 9 pour obtenir le composé de formule générale (2S)-I dans lequel le groupe R₁ représente l'atome d'hydrogène.

15. Procédé selon l'une quelconque des revendications 11 à 13 dans le cas où le groupe R₃ représente un groupe protecteur de l'amine et le groupe R₁ représente un groupe protecteur du groupe amine ou un groupe de formule -COOR₂ dans laquelle le groupe R₂ représente un groupe alkyle en C₁-C₆, un groupe aryle ou aralkyle, **caractérisé en ce qu'**il comporte une étape supplémentaire h₂) d'hydrogénolyse catalytique du composé de formule (2S)-I dans laquelle le groupe R₁ représente un groupe protecteur du groupe amine ou un groupe de formule
- COOR₂ dans laquelle le groupe R₂ représente un groupe alkyle en C₁-C₆, un groupe aryle ou aralkyle pour obtenir le composé de formule générale (2S)-I dans laquelle le groupe R₁ représente l'atome d'hydrogène.

16. Procédé selon l'une quelconque des revendications 8, 11 à 15, **caractérisé en ce qu'**il comporte une étape supplémentaire de purification du composé de formule générale (2S)-I dans lequel le groupe R₁ représente l'atome d'hydrogène.

17. Procédé selon la revendication 16 **caractérisé en ce que** l'étape de purification consiste en la neutralisation avec un acide organique du composé de formule générale (2S)-I dans lequel le groupe R₁ représente l'atome d'hydrogène et sa cristallisation dans un alcool afin d'obtenir le composé de formule générale (2S)-1 dans lequel le groupe R₁ représente l'atome d'hydrogène pur.

18. Procédé de synthèse de composés de formule générale (2S)-I suivante : 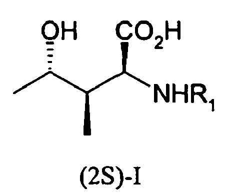 dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle
par hydrolyse diastéréosélective de la lactone de formule générale VI ou VII suivante : 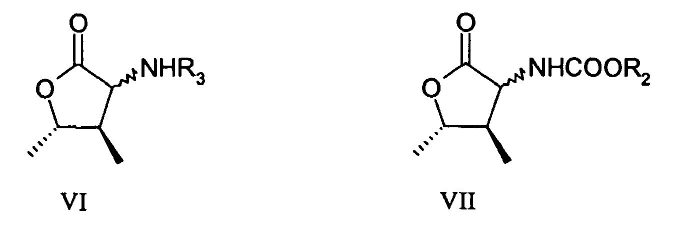 dans lesquelles le groupe **R₃** représente l'atome d'hydrogène ou un groupe protecteur du groupe amine et le groupe R₂ représente un groupe alkyle en C₁-C₆, un groupe aryle ou aralkyle, la lactone de formule générale VII étant obtenue par traitement de la lactone de formule générale VI,
ou par hydrolyse alcaline du sel de la lactone de formule 9 suivante : 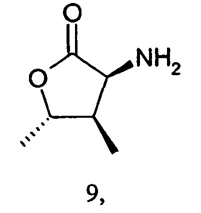 **caractérisée en ce que** la lactone de formule générale VI est obtenue par traitement avec un acide de l'aminonitrile de formule générale III suivante : 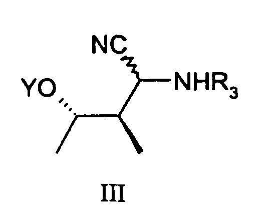 dans laquelle :
le groupe Y représente un groupe protecteur du groupe OH
et le groupe R₃ représente un groupe protecteur du groupe amine
et le sel de lactone de formule 9 est obtenu par traitement avec un acide de l'aminonitrile de formule générale V suivante : 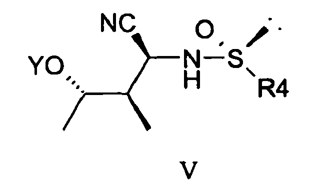 dans laquelle :
le groupe Y représente un groupe protecteur du groupe OH
et le groupe R4 représente un groupe alkyle en C₁-C₆, aryle ou aralkyle.

19. Procédé selon la revendication 18 dans le cas où le groupe R₁ représente un groupe protecteur du groupe amine ou un groupe de formule -COOR₂ dans laquelle le groupe R₂ représente un groupe alkyle en C₁-C₆, un groupe aryle ou aralkyle, **caractérisé en ce que** l'hydrolyse diastéréosélective consiste dans :
- L'hydrolyse alcaline de la lactone de formule générale VII ou VI dans laquelle R₃ représente un groupe protecteur du groupe amine pour obtenir un mélange de diastéréomères de formule générale 1 suivante : 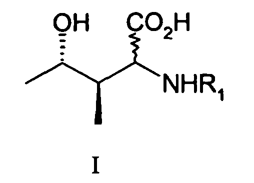
dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
- L'ajout d'acide organique, de préférence TFA, sous forme de traces, et le chauffage pour recycliser le composé de formule générale (2R)-I suivante : 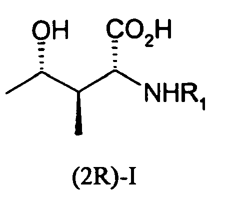
dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
en lactone de formule générale (3R)-VIII suivante : 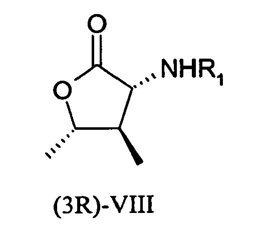 dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle,
- L'extraction avec un solvant organique de la lactone de formule générale (3R)-VIII pour récupérer le composé de formule générale (2S)-I dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle.

20. Procédé selon la revendication 18, **caractérisé en ce que** l'hydrolyse diastéréosélective consiste dans :
- L'hydrolyse enzymatique diastéréosélective de la lactone de formule générale (3R)-VI ou (3R)-VII pour donner le composé de formule générale (2R)-I,
- L'extraction avec un solvant organique de la lactone non hydrolysée restante de formule générale (3S)-VIII suivante : 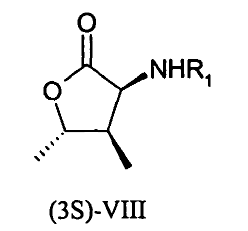
dans laquelle le groupe R₁ représente l'atome d'hydrogène, un groupe protecteur du groupe amine ou un groupe de formule -COOR₂ dans laquelle le groupe R₂ représente un groupe alkyle en C₁-C₆, un groupe aryle ou aralkyle,
- L'hydrolyse enzymatique non sélective de la lactone de formule générale (3S)-VIII pour obtenir le composé de formule générale (2S)-I.

21. Procédé selon la revendication 18 dans le cas où le groupe R₃ représente le groupe protecteur du groupe amine **caractérisé en ce que** l'hydrolyse diastéréosélective consiste dans :
- la cristallisation dans un alcool de la lactone de formule générale VI dans un solvant organique de façon à obtenir le sel du composé de formule (3S)-VIII dans laquelle le groupe R₁ représente un groupe protecteur du groupe amine,
- l'hydrogénolyse catalytique du sel du composé de formule (3S)-VIII dans laquelle le groupe R₁ représente un groupe protecteur du groupe amine de façon à obtenir le sel de la lactone de formule 9,
- l'hydrolyse alcaline, du sel de la lactone de formule 9 pour obtenir le composé de formule générale (2S)-I dans lequel le groupe R₁ représente l'atome d'hydrogène.

22. Procédé selon l'une quelconque des revendications 18 à 20 dans le cas où le groupe R₁ représente un groupe protecteur du groupe amine ou un groupe de formule -COOR₂ dans laquelle le groupe R₂ représente un groupe alkyle en C₁-C₆, un groupe aryle ou aralkyle, **caractérisé en ce qu'**il comporte une étape supplémentaire d'hydrogénolyse catalytique du composé de formule (2S)-I dans laquelle le groupe R₁ représente un groupe protecteur du groupe amine ou un groupe de formule -COOR₂ dans laquelle le groupe R₂ représente un groupe alkyle en C₁-C₆, un groupe aryle ou aralkyle pour obtenir le composé de formule générale (2S)-I dans laquelle le groupe R₁ représente l'atome d'hydrogène.

23. Procédé selon l'une quelconque des revendications 18 à 22, **caractérisé en ce que** l'aminonitrile de formule générale III est obtenu par traitement de l'aldéhyde de formule générale II suivante : 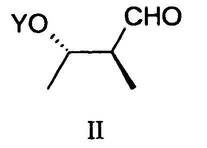 dans laquelle le groupe Y représente un groupe protecteur du groupe OH.

24. Procédé selon l'une quelconque des revendications 18 à 22, **caractérisé en ce que** l'aminonitrile de formule générale V est obtenu par traitement du composé de formule générale IV suivante : 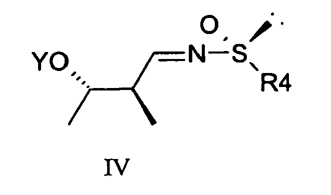 dans laquelle :
le groupe Y représente un groupe protecteur du groupe OH
et le groupe R4 représente un groupe alkyle en C₁-C₆, aryle ou aralkyle.

25. Procédé selon la revendication 24, **caractérisé en ce que** le composé de formule générale IV est obtenu par traitement de l'aldéhyde de formule générale II avec une sulfinamide de configuration (S)-(+).

26. Procédé selon l'une quelconque des revendications 23 et 25, **caractérisé en ce que** l'aldéhyde de formule générale **II** est obtenu par les étapes de :
- protection par un groupe Y du groupe OH de l'alcool de formule 3 suivante : 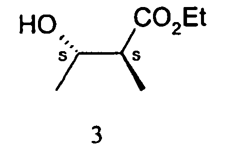
- réduction du groupe -COOEt en alcool et
- oxydation du groupe OH non protégé obtenu à l'étape précédente.

27. Procédé selon la revendication 26, **caractérisé en ce que** l'alcool de formule 3 est obtenu avec un excès énantiomérique d'au moins 85%, de préférence d'au moins 90%, et un excès diastéréomérique d'au moins 90 %, de préférence d'au moins 95% par la réduction diastéréo et énantiosélective du composé de formule 2 suivante :

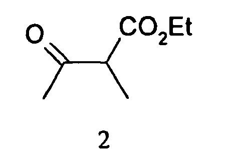

28. Procédé selon la revendication 27, **caractérisé en ce que** la réduction diastéréo et énantiosélective se fait par utilisation d'une enzyme.

29. Procédé selon la revendication 28, **caractérisé en ce que** l'on utilise un micro-organisme contenant cette enzyme, en particulier le *Geotrichum candidum.*

30. Lactone de formule générale VIII suivante : 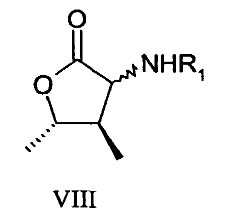 dans laquelle le groupe R₁ représente un groupe de formule -COOR₂ dans laquelle le groupe R₂ représente un groupe alkyle en C₁-C₆, un groupe aryle ou aralkyle sous forme libre ou sous forme de sels.

31. Aminonitrile de formule générale III suivante : 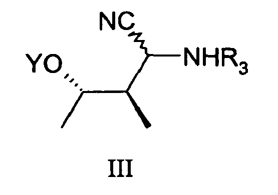 dans laquelle :
le groupe Y représente un groupe protecteur du groupe OH
et le groupe R₃ représente un groupe protecteur du groupe amine.

32. Aminonitrile selon la revendication 31, **caractérisé en ce qu'**il est représenté par la formule générale V suivante : 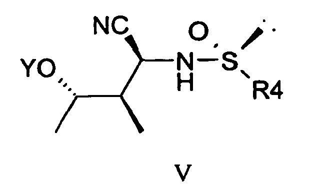 dans laquelle :
le groupe Y représente un groupe protecteur du groupe OH
et le groupe R4 représente un groupe alkyle en C₁-C₆, aryle ou aralkyle.

33. Composé de formule générale formule générale IV suivante : 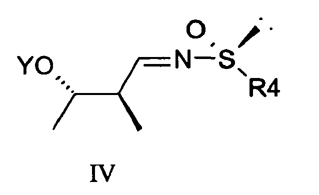 dans laquelle :
le groupe Y représente un groupe protecteur du groupe OH
et le groupe R4 représente un groupe alkyle en C₁-C₆, aryle ou aralkyle.

34. Composé de formule générale I suivante : 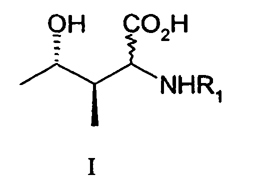 dans laquelle le groupe R₁ représente un groupe de formule -COOR₂ dans laquelle le groupe R₂ représente un groupe alkyle en C₁-C₆, un groupe aryle ou aralkyle.

## Claims

1. Method for synthesizing compounds of the following general formula (2S)-1: 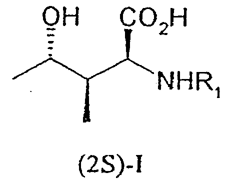 in which the group R₁ represents a hydrogen atom, a group protecting the amine group or a group of formula -COOR₂ in which the group R₂ represents a C₁-C₆ alkyl group, an aryl or aralkyl group
comprising the step a) of diastereo- and enantioselective reduction of the compound of the following formula 2: 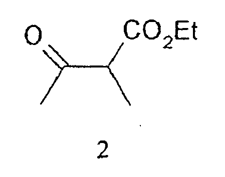 in order to obtain the compound of the following formula 3: 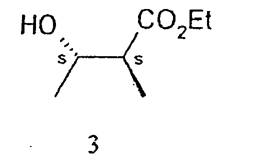 with an enantiomeric excess of at least 85%, preferably of at least 90%, and a diastereomeric excess of at least 90%, preferably of at least 95%.

2. Method according to Claim 1, **characterized in that** the diastereo- and enantioselective reduction is carried out using an enzyme.

3. Method according to Claim 2, **characterized in that** a microorganism containing this enzyme, in particular *Geotrichum candidum,* is used.

4. Method according to any one of the preceding claims, **characterized in that** it comprises the additional steps of:
b) protection of the OH group of the alcohol of formula 3 with a group Y,
c) reduction of the group -COOEt to an alcohol, and
d) oxidation of the unprotected OH group obtained in
c) to obtain the aldehyde of the following general formula II: 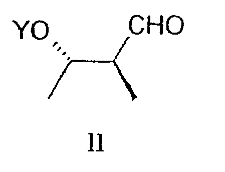 in which the group Y represents a group protecting the OH group.

5. Method according to Claim 4, **characterized in that** it comprises an additional step e₁) of treatment of the compound of general formula II with a sulfinamide having the configuration (S)(+) in order to obtain the compound of the following general formula IV: 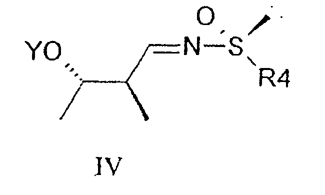 in which
the group Y represents a group protecting the OH group and the group R4 represents a C₁-C₆ alkyl, aryl or aralkyl group.

6. Method according to Claim 5, **characterized in that** it comprises an additional step f₁) of treatment of the compound of general formula IV in order to obtain the aminonitrile of the following general formula V: 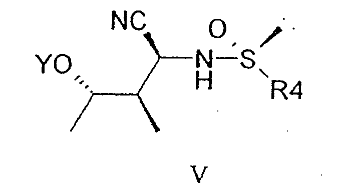 in which
the group Y represents a group protecting the OH group
and the group R4 represents a C₁-C₆ alkyl, aryl or aralkyl group.

7. Method according to Claim 6, **characterized in that** it comprises an additional step g₁) of treatment with an acid of the aminonitrile of general formula V in order to obtain the salt of the lactone of the following formula 9:

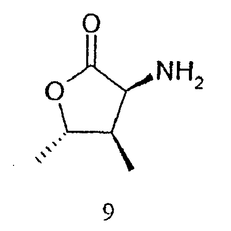

8. Method according to Claim 7, **characterized in that** it comprises an additional step h₁) of alkaline hydrolysis of the salt of the lactone of formula 9 in order to obtain the compound of general formula (2S)-I in which the group R₁ represents a hydrogen atom.

9. Method according to Claim 4, **characterized in that** it comprises an additional step e₂) of treatment of the compound of general formula II in order to obtain the aminonitrile of the following general formula III: 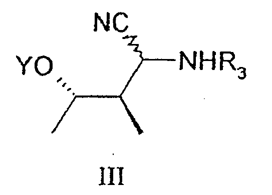 in which:
the group Y represents a group protecting the OH group and the group R₃ represents a group protecting the amine group.

10. Method according to Claim 9, **characterized in that** it comprises an additional step f₂) of treatment with an acid of the aminonitrile of general formula III in order to obtain the lactone of the following general formula VI: 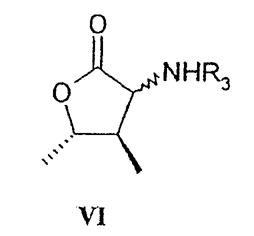 in which the group R₃ represents a hydrogen atom or a group protecting the amine group
followed, if necessary, by an additional step f_{2.1}) of treatment of the lactone of general formula VI in order to obtain the lactone of the following general formula VII: 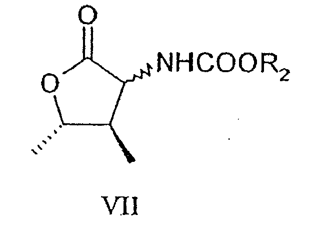 in which the group R₂ represents a C₁-C₆ alkyl group, an aryl or aralkyl group.

11. Method according to Claim 10, **characterized in that** it comprises an additional step g₂) of diastereoselective hydrolysis of the lactone of general formula VI or VII in order to obtain the compound of general formula (2S)-I.

12. Method according to Claim 11, in the case where the group R₃ represents the group protecting the amine group and the group R₁ represents a group protecting the amine group or a group of formula -COOR₂ in which the group R₂ represents a C₁-C₆ alkyl group, an aryl or aralkyl group, **characterized in that** the diastereoselective hydrolysis consists in:
- The alkaline hydrolysis of the lactone of general formula VI in which the group R₃ represents a group protecting the amine group or of general formula VII in order to obtain a mixture of diastereomers of the following general formula I: 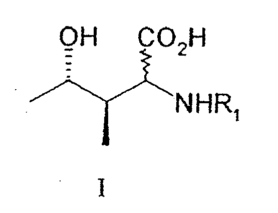
in which the group R₁ represents a group protecting the amine group or a group of formula -COOR₂ in which the group R₂ represents a C₁-C₆ alkyl group, an aryl or aralkyl group,
- The addition of an organic acid, preferably TFA, in the form of traces, and heating in order to recyclize the compound of the following general formula (2R) -I : 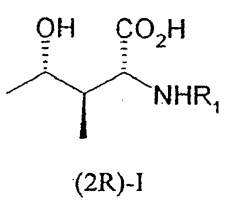
in which the group R₁ represents a group protecting the amine group or a group of formula -COOR₂ in which the group R₂ represents a C₁-C₆ alkyl group, an aryl or aralkyl group,
into a lactone of the following general formula (3R)-VIII: 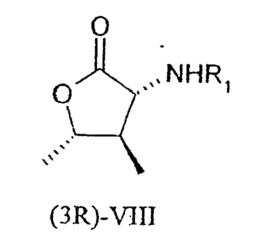 in which the group R₁ represents a group protecting the amine group or a group of formula -COOR₂ in which the group R₂ represents a C₁-C₆ alkyl group, an aryl or aralkyl group,
- The extraction, with an organic solvent, of the lactone of general formula (3R)-VIII in order to recover the compound of general formula (2S)-I in which the group R₁ represents a group protecting the amine group or a group of formula -COOR₂ in which the group R₂ represents a C₁-C₆ alkyl group, an aryl or aralkyl group.

13. Method according to Claim 11, **characterized in that** the diastereoselective hydrolysis consists in:
- The diastereoselective enzymatic hydrolysis of the lactone of general formula (3R)-VI or (3R)-VII to give the compound of general formula (2R)-I,
- The extraction with an organic solvent of the remaining nonhydrolyzed lactone of the following general formula (3S)-VIII: 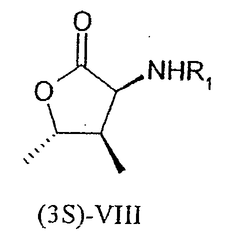
in which the group R₁ represents a hydrogen atom, a group protecting the amine group or a group of formula
- COOR₂ in which the group R₂ represents a C₁-C₆ alkyl group, an aryl or aralkyl group,
- The nonselective enzymatic hydrolysis of the lactone of general formula (3S)-VIII in order to obtain the compound of general formula (2S)-I in which the group R₁ represents a hydrogen atom, a group protecting the amine group or a group of formula -COOR₂ in which the group R₂ represents a C₁-C₆ alkyl group, an aryl or aralkyl group.

14. Method according to Claim 11, in the case where the group R₃ represents the group protecting the amine group, **characterized in that** the diastereoselective hydrolysis consists in:
- the crystallization, from an alcohol, of the lactone of general formula VI in an organic solvent so as to obtain the salt of the compound of formula (3S)-VIII in which the group R₁ represents a group protecting the amine group,
- the catalytic hydrogenolysis of the salt of the compound of formula (3S)-VIII in which the group R₁ represents a group protecting the amine group so as to obtain the salt of the lactone of formula 9,
- the alkaline hydrolysis of the salt of the lactone of formula 9 in order to obtain the compound of general formula (2S)-I in which the group R₁ represents a hydrogen atom.

15. Method according to any one of Claims 11 to 13 in the case where the group R₃ represents a group protecting the amine and the group R₁ represents a group protecting the amine group or a group of formula -COOR₂ in which the group R₂ represents a C₁-C₆ alkyl group, an aryl or aralkyl group, **characterized in that** it comprises an additional step h₂) of catalytic hydrogenolysis of the compound of formula (2S)-I in which the group R₁ represents a group protecting the amine group or a group of formula -COOR₂ in which the group R₂ represents a C₁-C₆ alkyl group, an aryl or aralkyl group in order to obtain the compound of general formula (2S)-I in which the group R₁ represents a hydrogen atom.

16. Method according to any one of Claims 8, 11 to 15, **characterized in that** it comprises an additional step of purifying the compound of general formula (28)-I in which the group R₁ represents a hydrogen atom.

17. Method according to Claim 16, **characterized in that** the purification step consists in neutralizing, with an organic acid, the compound of general formula (2S)-I in which the group R₁ represents a hydrogen atom and its crystallization from an alcohol in order to obtain the compound of general formula (2S)-I in which the group R₁ represents a hydrogen atom pure.

18. Method for synthesizing compounds of the following general formula (2S)-I: 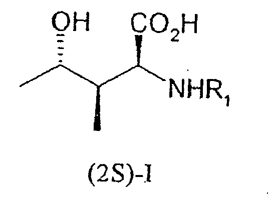 in which the group R₁ represents a hydrogen atom, a group protecting the amine group or a group of formula -COOR₂ in which the group R₂ represents a C₁-C₆ alkyl group, an aryl or aralkyl group
by diastereoselective hydrolysis of the lactone of the following general formula VI or VII: 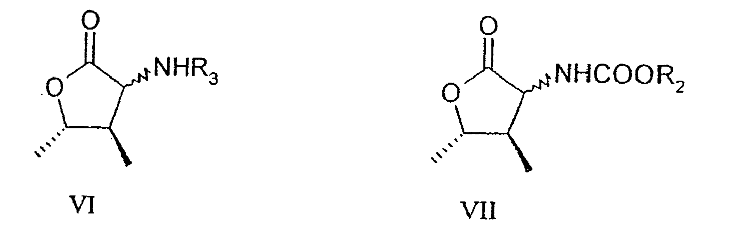 in which the group R₃ represents a hydrogen atom or a group protecting the amine group or the group R₂ represents a C₁-C₆ alkyl group, an aryl or aralkyl group, the lactone of general formula VII being obtained by treating the lactone of general formula VI,
or by alkaline hydrolysis of the salt of the lactone of the following formula 9: 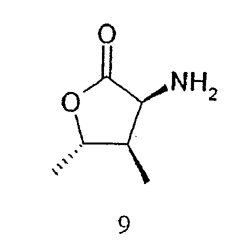 **characterized in that** the lactone of general formula VI is obtained by treating, with an acid, the aminonitrile of the following general formula III: 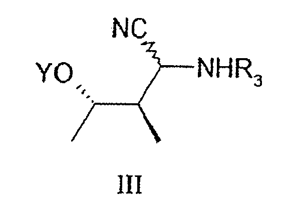 in which:
the group Y represents a group protecting the OH group and the group R₃ represents a group protecting the amine group,
the lactone salt of formula 9 is obtained by treating, with an acid, the aminonitrile of the following general formula V: 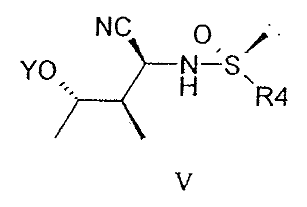 in which:
the group Y represents a group protecting the OH group
and the group R4 represents a C₁-C₆ alkyl, aryl or aralkyl group.

19. Method according to Claim 18, in the case where the group R₁ represents a group protecting the amine group or a group of formula -COOR₂ in which the group R₂ represents a C₁-C₆ alkyl group, an aryl or aralkyl group, **characterized in that** the diastereoselective hydrolysis consists in:
- The alkaline hydrolysis of the lactone of general formula VII or VI in which R₃ represents a group protecting the amine group in order to obtain a mixture of diastereoisomers of the following general formula I: 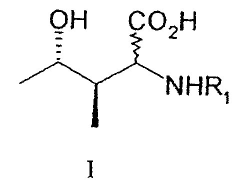
in which the group R₁ represents a group protecting the amine group or a group of formula -COOR₂ in which the group R₂ represents a C₁-C₆ alkyl group, an aryl or aralkyl group,
- The addition of an organic acid, preferably TFA, in the form of traces, and heating in order to recyclize the compound of the following general formula (2R)-I : 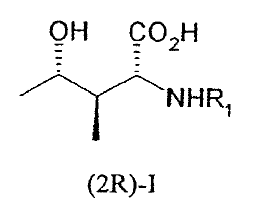
in which the group R₁ represents a group protecting the amine group or a group of formula -COOR₂ in which the group R₂ represents a C₁-C₆ alkyl group, an aryl or aralkyl group,
into a lactone of the following general formula (3R)-VIII: 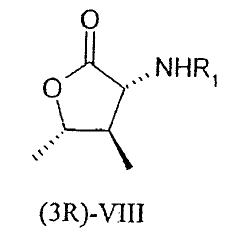 in which the group R₁ represents a group protecting the amine group or a group of formula -COOR₂ in which the group R₂ represents a C₁-C₆ alkyl group, an aryl or aralkyl group,
- The extraction, with an organic solvent, of the lactone of general formula (3R)-VIII in order to recover the compound of general formula (2S)-I in which the group R₁ represents a group protecting the amine group or a group of formula -COOR₂ in which the group R₂ represents a C₁-C₆ alkyl group, an aryl or aralkyl group.

20. Method according to Claim 18, **characterized in that** the diastereoselective hydrolysis consists in:
- The diastereoselective enzymatic hydrolysis of the lactone of general formula (3R) -VI or (3R)-VII to give the compound of general formula (2R)-I,
- The extraction with an organic solvent of the remaining nonhydrolyzed lactone of the following general formula (3S)-VIII: 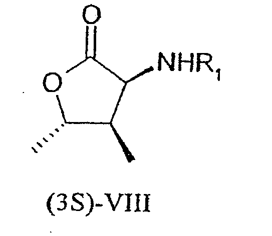
in which the group R₁ represents a hydrogen atom, a group protecting the amine group or a group of formula
- COOR₂ in which the group R₂ represents a C₁-C₆ alkyl group, an aryl or aralkyl group,
- The nonselective enzymatic hydrolysis of the lactone of general formula (3S)-VIII in order to obtain the compound of general formula (2S)-I.

21. Method according to Claim 18, in the case where the group R₃ represents the group protecting the amine group, **characterized in that** the diastereoselective hydrolysis consists in:
- the crystallization, from an alcohol, of the lactone of general formula VI in an organic solvent so as to obtain the salt of the compound of formula (3S)-VIII in which the group R₁ represents a group protecting the amine group,
- the catalytic hydrogenolysis of the salt of the compound of formula (3S)-VIII in which the group R₁ represents a group protecting the amine group so as to obtain the salt of the lactone of formula 9,
- the alkaline hydrolysis of the salt of the lactone of formula 9 in order to obtain the compound of
general formula (2S)-I in which the group R₁ represents a hydrogen atom.

22. Method according to any one of Claims 18 to 20 in the case where the group R₁ represents a group protecting the amine group or a group of formula -COOR₂ in which the group R₂ represents a C₁-C₆ alkyl group, an aryl or aralkyl group, **characterized in that** it comprises an additional step of catalytic hydrogenolysis of the compound of formula (2S)-I in which the group R₁ represents a group protecting the amine group or a group of formula -COOR₂ in which the group R₂ represents a C₁-C₆ alkyl group, an aryl or aralkyl group in order to obtain the compound of general formula (2S)-I in which the group R₁ represents a hydrogen atom.

23. Method according to any one of Claims 18 to 22, **characterized in that** the aminonitrile of general formula III is obtained by treating the aldehyde of the following general formula II: 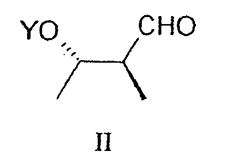 in which the group Y represents a group protecting the OH group.

24. Method according to any one of Claims 18 to 22, **characterized in that** the aminonitrile of general formula V is obtained by treating the compound of the following general formula IV: 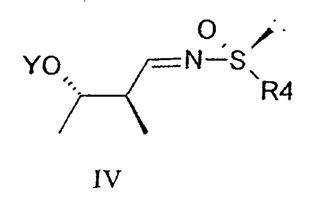 in which:
the group Y represents a group protecting the OH group and the group R4 represents a C₁-C₆ alkyl, aryl or aralkyl group.

25. Method according to Claim 24, **characterized in that** the compound of general formula IV is obtained by treating the aldehyde of general formula II with a sulfinamide having the configuration (S)-(+).

26. Method according to either of Claims 23 and 25, **characterized in that** the aldehyde of general formula II is obtained by the steps of:
- protection, with a group Y, of the OH group of the alcohol of the following formula 3: 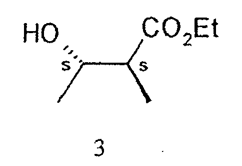
- reduction of the -COOEt group to an alcohol and
- oxidation of the unprotected OH group obtained in the preceding step.

27. Method according to Claim 26, **characterized in that** the alcohol of formula 3 is obtained with an enantiomeric excess of at least 85%, preferably of at least 90%, and a diastereomeric excess of at least 90%, preferably of at least 95% by diastereo- and enantioselective reduction of the compound of the following formula 2:

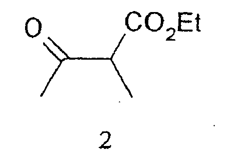

28. Method according to Claim 27, **characterized in that** the diastereo- and enantioselective reduction is carried out using an enzyme.

29. Method according to Claim 28, **characterized in that** a microorganism containing this enzyme, in particular *Geotrichum candidum,* is used.

30. Lactone of the following general formula VIII: 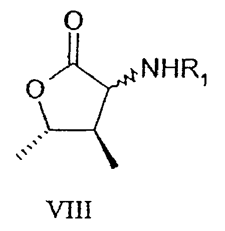 in which the group R₁ represents a group of formula -COOR₂ in which the group R₂ represents a C₁-C₆ alkyl group, an aryl or aralkyl group
in free form or in the form of salts.

31. Aminonitrile of the following general formula III: 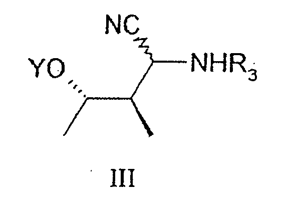 in which:
the group Y represents a group protecting the OH group and the group R₃ represents a group protecting the amine group.

32. Aminonitrile according to Claim 31,
**characterized in that** it is represented by the following general formula V: 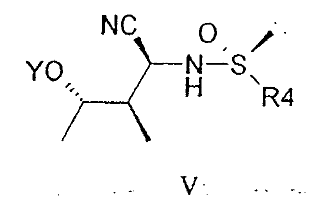 in which:
the group Y represents a group protecting the OH group and the group R4 represents a C₁-C₆ alkyl, aryl or aralkyl group.

33. Compound of the following general formula IV: 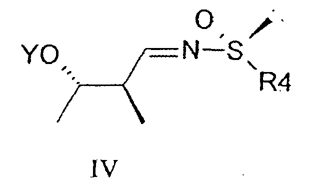 in which:
the group Y represents a group protecting the OH group and the group R4 represents a C₁-C₆ alkyl, aryl or aralkyl group.

34. Compound of the following general formula I: 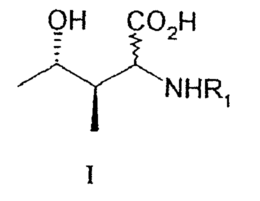 in which the group R₁ represents a group of formula -COOR₂ in which the group R₂ represents a C₁-C₆ alkyl group, an aryl or aralkyl group.

## Patentansprüche

1. Verfahren zur Synthese von Verbindungen der folgenden allgemeinen Formel (2S)-I: 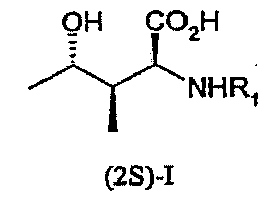 in der die Gruppe R₁ ein Wasserstoffatom, eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
umfassend den Schritt (a) der diastereo- und enantioselektiven Reduktion der Verbindung der folgenden Formel 2: 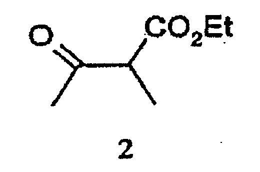 um die folgende Verbindung der Formel 3: 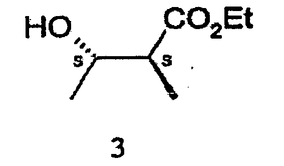 mit einem enantiomeren Überschuss von mindestens 85 %, bevorzugt mindestens 90 %, und einem diastereomeren Überschuss von mindestens 90 %, bevorzugt mindestens 95 % zu erhalten.

2. Verfahren nach Anspruch 1, **dadurch gekennzeichnet, dass** die diastereo- und enantioselektive Reduktion unter Verwendung eines Enzyms vorgenommen wird.

3. Verfahren nach Anspruch 2, **dadurch gekennzeichnet, dass** man einen Mikroorganismus, der dieses Enzym enthält, insbesondere *Geotrichum candidum,* verwendet.

4. Verfahren nach irgendeinem der vorangehenden Ansprüche, **dadurch gekennzeichnet, dass** es die zusätzlichen Schritte umfasst:
b) Schützen der OH-Gruppe des Alkohols der Formel 3 durch eine Gruppe Y,
c) Reduktion der Gruppe -COOEt zu einem Alkohol und
d) Oxidation der nicht geschützten OH-Gruppe, die in c) erhalten wird, um den Aldehyd der folgenden allgemeinen Formel II zu erhalten: 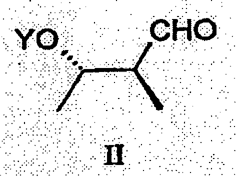 in der die Gruppe Y eine Schutzgruppe der OH-Gruppe darstellt.

5. Verfahren nach Anspruch 4, **dadurch gekennzeichnet, dass** es einen zusätzlichen Schritt e₁) der Behandlung der Verbindung der allgemeinen Formel II mit einem Sulfinamid mit (S)(+)-Konfiguration umfasst, um die Verbindung der folgenden allgemeinen Formel IV zu erhalten, 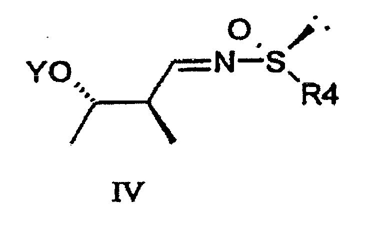 in der
die Gruppe Y eine Schutzgruppe der OH-Gruppe darstellt und
die Gruppe R4 eine (C₁ - C₆)-Alkyl-, Aryl- oder Aralkylgruppe darstellt.

6. Verfahren nach Anspruch 5, **dadurch gekennzeichnet, dass** es einen zusätzlichen Schritt f₁) der Behandlung der Verbindung der allgemeinen Formel IV umfasst, um das Aminonitril der folgenden allgemeinen Formel V zu erhalten: 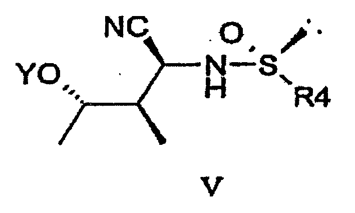 in der
die Gruppe Y eine Schutzgruppe der OH-Gruppe darstellt und
die Gruppe R4 eine (C1 - C6)-Alkyl-, Aryl- oder Aralkylgruppe darstellt.

7. Verfahren nach Anspruch 6, **dadurch gekennzeichnet, dass** es einen zusätzlichen Schritt g₁) der Behandlung des Aminonitrils der allgemeinen Formel V mit einer Säure umfasst, um das Salz des Lactons der folgenden Formel 9 zu erhalten:

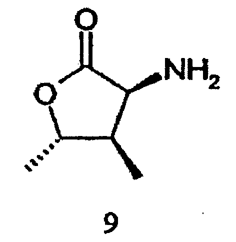

8. Verfahren nach Anspruch 7, **dadurch gekennzeichnet, dass** es einen zusätzlichen Schritt h₁) der alkalischen Hydrolyse des Salzes des Lactons der Formel 9 umfasst, um die Verbindung der allgemeinen Formel (2S)-I zu erhalten, in der die Gruppe R₁ ein Wasserstoffatom darstellt.

9. Verfahren nach Anspruch 4, **dadurch gekennzeichnet, dass** es einen zusätzlichen Schritt e₂) der Behandlung der Verbindung der allgemeinen Formel II umfasst, um das Aminonitril der folgenden allgemeinen Formel III zu erhalten: 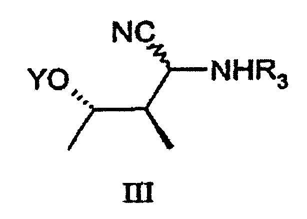 in der
die Gruppe Y eine Schutzgruppe der OH-Gruppe darstellt und
die Gruppe R₃ eine Schutzgruppe der Amingruppe darstellt.

10. Verfahren nach Anspruch 9, **dadurch gekennzeichnet, dass** es einen zusätzlichen Schritt f₂) der Behandlung des Aminonitrils der allgemeinen Formel III mit einer Säure umfasst, um das Lacton der folgenden allgemeinen Formel VI zu erhalten: 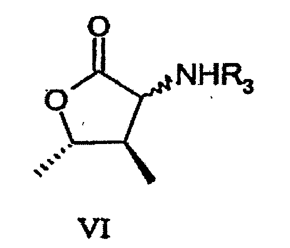 in der die Gruppe R₃ ein Wasserstoffatom oder eine Schutzgruppe der Amingruppe darstellt,
gefolgt, falls erforderlich, von einem zusätzlichen Schritt f_{2,1}) der Behandlung des Lactons der allgemeinen Formel VI, um das Lacton der folgenden allgemeinen Formel VII zu erhalten: 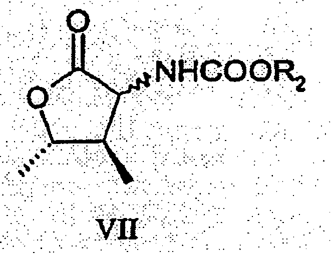 in der die Gruppe R₂ eine (C₁-C₆)-Alkylgruppe, eine Aryl- oder Aralkylgruppe darstellt.

11. Verfahren nach Anspruch 10, **dadurch gekennzeichnet, dass** es einen zusätzlichen Schritt g₂) der diastereoselektiven Hydrolyse des Lactons der allgemeinen Formel VI oder VII umfasst, um die Verbindung der allgemeinen Formel (2S)-I zu erhalten.

12. Verfahren nach Anspruch 11 für den Fall, in dem die Gruppe R₃ die Schutzgruppe der Amingruppe darstellt und die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, **dadurch gekennzeichnet, dass** die diastereoselektive Hydrolyse besteht aus:
- der alkalischen Hydrolyse des Lactons der allgemeinen Formel VI, in der die Gruppe R₃ eine Schutzgruppe der Amingruppe darstellt, oder der allgemeinen Formel VII, um eine Mischung von Diastereomeren der folgenden allgemeinen Formel I zu erhalten: 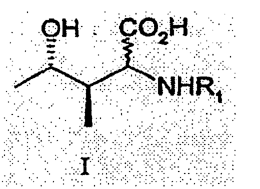 in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
- der Zugabe von organischer Säure, bevorzugt TFA, in Form von Spuren und Erwärmen, um die Verbindung der folgenden allgemeinen Formel (2R)-I: 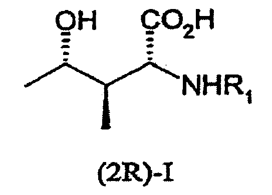 in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
zum Lacton der folgenden allgemeinen Formel (3R)-VIII zu recyclisieren: 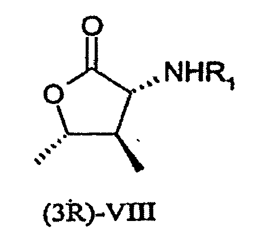 in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
- der Extraktion des Lactons der allgemeinen Formel (3R)-VIII mit einem organischen Lösungsmittel, um die Verbindung der allgemeinen Formel (2S)-I zu gewinnen, in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt.

13. Verfahren nach Anspruch 11, **dadurch gekennzeichnet, dass** die diastereoselektive Hydrolyse besteht aus:
- der diastereoselektiven enzymatischen Hydrolyse des Lactons der allgemeinen Formel (3R)-VI oder (3R)-VII, um die Verbindung der allgemeinen Formel (2R)-I zu ergeben,
- der Extraktion des verbleibenden nicht hydrolysierten Lactons der folgenden allgemeinen Formel (3S)-VIII mit einem organischen Lösungsmittel: 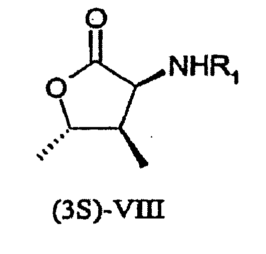 in der die Gruppe R₁ ein Wasserstoffatom, eine Schutzgruppe der Amingruppe oder eine Gruppe -COOR₂ darstellt, worin die Gruppe R₂ eine (C₁ - C₆)-Alkylgruppe, eine Aryl- oder Aralkylgruppe darstellt,
- der nicht-selektiven enzymatischen Hydrolyse des Lactons der allgemeinen Formel (3S)-VIII, um die Verbindung der allgemeinen Formel (2S)-1 zu erhalten, in der die Gruppe R₁ ein Wasserstoffatom, eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt.

14. Verfahren nach Anspruch 11 für den Fall, in dem R₃ die Schutzgruppe der Amingruppe darstellt, **dadurch gekennzeichnet, dass** die diastereoselektive Hydrolyse besteht aus:
- der Kristallisation in einem Alkohol des Lactons der allgemeinen Formel VI in einem organischen Lösungsmittel auf solche Weise, dass man das Salz der Verbindung der Formel (3S)-VIII erhält, in der die Gruppe R₁ eine Schutzgruppe der Amingruppe darstellt,
- der katalytischen Hydrogenolyse des Salzes der Verbindung der Formel (3S)-VIII, in der die Gruppe R₁ eine Schutzgruppe der Amingruppe darstellt, um das Salz des Lactons der Formel 9 zu erhalten,
- der alkalischen Hydrolyse des Salzes des Lactons der Formel 9, um die Verbindung der allgemeinen Formel (2S)-I zu erhalten, in der die Gruppe R₁ ein Wasserstoffatom darstellt.

15. Verfahren nach irgendeinem der Ansprüche 11 bis 13 für den Fall, in dem die Gruppe R₃ eine Schutzgruppe des Amins darstellt und die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, **dadurch gekennzeichnet, dass** es einen zusätzlichen Schritt h₂) der katalytischen Hydrogenolyse der Verbindung der Formel (2S)-I umfasst, in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, um die Verbindung der allgemeinen Formel (2S)-I zu erhalten, in der die Gruppe R₁ ein Wasserstoffatom darstellt.

16. Verfahren nach irgendeinem der Ansprüche 8, 11 bis 15, **dadurch gekennzeichnet, dass** es einen zusätzlichen Schritt der Reinigung der Verbindung der allgemeinen Formel (2S)-I umfasst, in der die Gruppe R₁ ein Wasserstoffatom darstellt.

17. Verfahren nach Anspruch 16, **dadurch gekennzeichnet, dass** der Reinigungsschritt aus der Neutralisation der Verbindung allgemeinen Formel (2S)-I, in der die Gruppe R₁ ein Wasserstoffatom darstellt, mit einer organischen Säure und ihrer Kristallisation in einem Alkohol besteht, um die Verbindung der allgemeinen Formel (2S)-I zu erhalten, in der die Gruppe R₁ ein reines Wasserstoffatom darstellt.

18. Verfahren zur Synthese von Verbindungen der folgenden allgemeinen Formel (2S)-I: 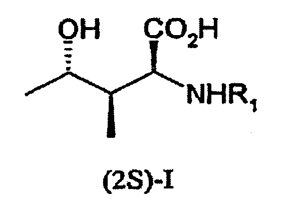 in der die Gruppe R₁ ein Wasserstoffatom, eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
durch diastereoselektive Hydrolyse des Lactons der folgenden allgemeinen Formel VI oder VII: 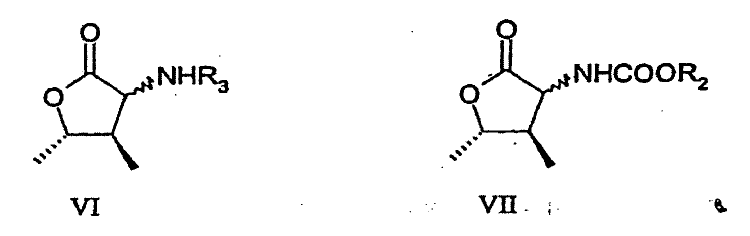 worin die Gruppe R₃ ein Wasserstoffatom oder eine Schutzgruppe der Amingruppe darstellt und die Gruppe R₂ eine (C₁ - C₆)-Alkylgruppe, eine Aryl- oder Aralkylgruppe darstellt, wobei das Lacton der allgemeinen Formel VII durch Behandlung des Lactons der allgemeinen Formel VI erhalten wird,
oder durch alkalische Hydrolyse des Salzes des Lactons der folgenden Formel 9: 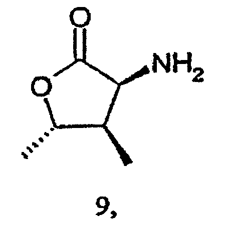 **dadurch gekennzeichnet, dass** das Lacton der allgemeinen Formel VI durch Behandlung des Aminonitrils der folgenden allgemeinen Formel III mit einer Säure erhalten wird: 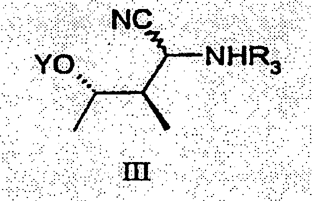 in der:
die Gruppe Y eine Schutzgruppe der OH-Gruppe darstellt und
die Gruppe R₃ eine Schutzgruppe der Amingruppe darstellt,
und das Salz des Lactons der Formel 9 durch Behandlung des Aminonitrils der folgenden allgemeinen Formel V mit einer Säure erhalten wird: 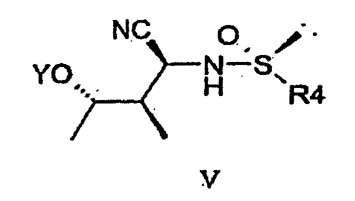
in der:
die Gruppe Y eine Schutzgruppe der QH-Gruppe darstellt und
die Gruppe R₄ eine (C₁- C₆)-Alkyl-, Aryl- oder Aralkylgruppe darstellt.

19. Verfahren nach Anspruch 18 für den Fall, in dem die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, **dadurch gekennzeichnet, dass** die diastereoselektive Hydrolyse besteht aus:
- der alkalischen Hydrolyse des Lactons der allgemeinen Formel VII oder VI, worin R₃ eine Schutzgruppe der Amingruppe darstellt, um eine Mischung von Diastereomeren der folgenden allgemeinen Formel I zu erhalten: 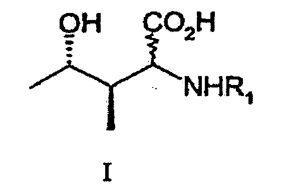 in der die Gruppe R₁ eine Schutzgruppe der Amingruppe oder eine Gruppe der Formel -COOR₂ darstellt, worin die Gruppe R₂ eine (C₁ - C₆)- Alkylgruppe, eine Aryl- oder Aralkylgruppe darstellt,
- der Zugabe von organischer Säure, bevorzugt von TFA in Form von Spuren und Erwärmen, um die Verbindung der allgemeinen Formel (2R)-I: 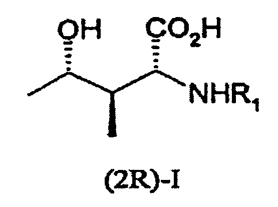 in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
zum Lacton der folgenden allgemeinen Formel (3R)-VIII zu recyclisieren: 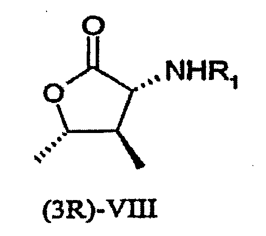 in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
- der Extraktion des Lactons der allgemeinen Formel (3R)-VIII mit einem organischen Lösungsmittel, um die Verbindung der allgemeinen Formel (2S)-1 zu gewinnen, in der die Gruppe R₁ eine Schutzgruppe der Amingruppe oder eine Gruppe der Formel -COOR₂ darstellt, worin die Gruppe R₂ eine (C₁ - C₆)-Akylgruppe, eine Aryl- oder Aralkylgruppe darstellt.

20. Verfahren nach Anspruch 18, **dadurch gekennzeichnet, dass** die diastereoselektive Hydrolyse besteht aus:
- der diastereoselektiven enzymatischen Hydrolyse des Lactons der allgemeinen Formel (3R)-VI oder (3R)-VII, um die Verbindung der allgemeinen Formel (2R)-I zu ergeben,
- der Extraktion des verbleibenden nicht hydrolysierten Lactons der folgenden allgemeinen Formel (3S)-VIII mit einem organischen Lösungsmittel: 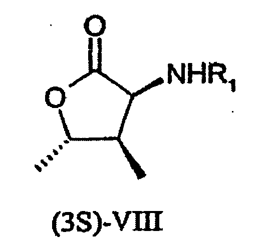 in der die Gruppe R₁ ein Wasserstoffatom, eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt,
- der nicht-selektiven enzymatischen Hydrolyse des Lactons der allgemeinen Formel (3S)-VIII, um die Verbindung der allgemeinen Formel (2S)-I zu erhalten.

21. Verfahren nach Anspruch 18 für den Fall, in dem die Gruppe R₃ die Schutzgruppe der Amingruppe darstellt, **dadurch gekennzeichnet, dass** die diastereoselektive Hydrolyse besteht aus:
- der Kristallisation in einem Alkohol des Lactons der allgemeinen Formel VI in einem organischen Lösungsmittel auf solche Weise, dass man das Salz der Verbindung der Formel (3S)-VIII erhält, in der die Gruppe R₁ eine Schutzgruppe der Amingruppe darstellt,
- der katalytischen Hydrogenolyse des Salzes der Verbindung der Formel (3S)-VIII, in der die Gruppe R₁ eine Schutzgruppe der Amingruppe darstellt, auf solche Weise, dass man das Salz des Lactons der Formel 9 erhält,
- der alkalischen Hydrolyse des Salzes des Lactons der Formel 9, um die Verbindung der allgemeinen Formel (2S)-I zu erhalten, in der die Gruppe R₁ ein Wasserstoffatom darstellt.

22. Verfahren nach irgendeinem der Ansprüche 18 bis 20 für den Fall, in dem die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, **dadurch gekennzeichnet, dass** es einen zusätzlichen Schritt der katalytischen Hydrogenolyse der Verbindung der Formel (2S)-I umfasst, in der die Gruppe R₁ eine Schutzgruppe der Amingruppe oder eine Gruppe der Formel -COOR₂ darstellt, worin die Gruppe R₂ eine (C₁ - C₆)-Alkylgruppe, eine Aryl- oder Aralkylgruppe darstellt, um die Verbindung der allgemeinen Formel (2S)-I zu erhalten, in der die Gruppe R₁ ein Wasserstoffatom darstellt.

23. Verfahren nach irgendeinem der Ansprüche 18 bis 22, **dadurch gekennzeichnet, dass** das Aminonitril der allgemeinen Formel III durch Behandlung-des Aldehyds der folgenden allgemeinen Formel II erhalten wird: 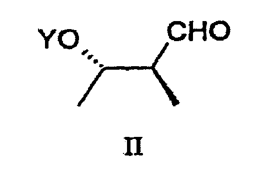 in der die Gruppe Y eine Schutzgruppe der OH-Gruppe darstellt.

24. Verfahren nach irgendeinem der Ansprüche 18 bis 22, **dadurch gekennzeichnet, dass** das Aminonitril der allgemeinen Formel V durch Behandlung der Verbindung der folgenden allgemeinen Formel IV erhalten wird: 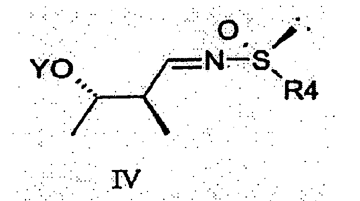 in der:
die Gruppe Y eine Schutzgruppe der OH-Gruppe darstellt und
die Gruppe R4 eine (C₁ - C₆)-Alkyl-, Aryl- oder Aralkylgruppe darstellt.

25. Verfahren nach Anspruch 24, **dadurch gekennzeichnet, dass** die Verbindung der allgemeinen Formel IV durch Behandlung des Aldehyds der allgemeinen Formel II mit einem Sulfinamid der Konfiguration (S)-(+) erhalten wird.

26. Verfahren nach irgendeinem der Ansprüche 23 und 25, **dadurch gekennzeichnet, dass** der Aldehyd der allgemeinen Formel II erhalten wird durch die Schritte:
- Schützen der OH-Gruppe des Alkohols der folgenden Formel 3: 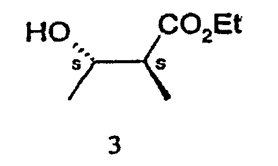 mit einer Gruppe Y,
- Reduktion der Gruppe -COOEt zum Alkohol und
- Oxidation der nicht geschützten OH-Gruppe, die im vorstehenden " Schritt erhalten wurde.

27. Verfahren nach Anspruch 26, **dadurch gekennzeichnet, dass** der Alkohol der Formel 3 mit einem enantiomeren Überschuss von mindestens 85 %, vorzugsweise mindestens 90 %, und einem diastereomeren Überschuss von mindestens 90 %, vorzugsweise mindestens 95 %, durch die diastereo- und enantioselektive Reduktion der Verbindung der folgenden Formel 2: 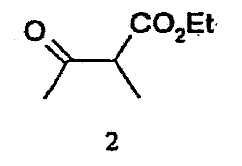 erhalten wird.

28. Verfahren nach Anspruch 27, **dadurch gekennzeichnet, dass** die diastereo- und enantioselektive Reduktion unter Verwendung eines Enzyms vorgenommen wird.

29. Verfahren nach Anspruch 28, **dadurch gekennzeichnet, dass** man einen Mikroorganismus, der dieses Enzym enthält, insbesondere *Geotrichum candidum* verwendet.

30. Lacton der folgenden allgemeinen Formel VIII: 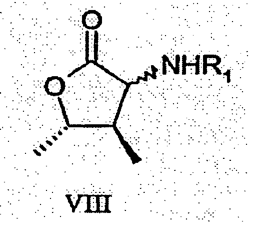 in der die Gruppe R₁ eine Gruppe der Formel -COOR₂ darstellt, worin die Gruppe R₂ eine (C₁ - C₆)-Alkylgruppe, eine Aryl- oder Aralkylgruppe darstellt, in freier Form oder in Form von Salzen.

31. Aminonitril der folgenden allgemeinen Formel III: 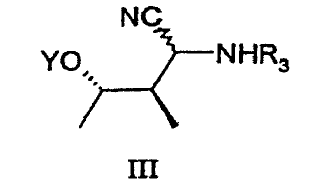 in der:
die Gruppe Y eine Schutzgruppe der OH-Gruppe darstellt und
die Gruppe R₃ eine Schutzgruppe der Amingruppe darstellt.

32. Aminonitril nach Anspruch 31, **dadurch gekennzeichnet, dass** es durch die folgende allgemeine Formel V dargestellt wird: 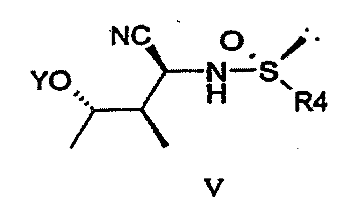 in der:
die Gruppe Y eine Schutzgruppe der OH-Gruppe darstellt und
die Gruppe R4 eine (C₁ - C₆)-Alkyl-, Aryl- oder Aralkylgruppe darstellt.

33. Verbindung der folgenden allgemeinen Formel IV: 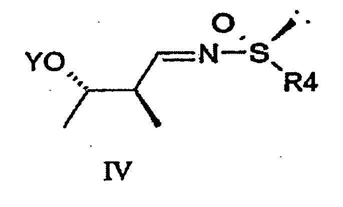 in der
die Gruppe Y eine Schutzgruppe der OH-Gruppe darstellt und
die Gruppe R₄ eine (C₁- C₆)-Alkyl-, Aryl- oder Aralkylgruppe darstellt.

34. Verbindung der folgenden allgemeinen Formel I: 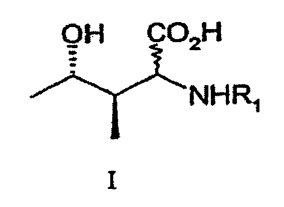 in der die Gruppe R₁ eine Gruppe der Formel -COOR₂ darstellt, worin die Gruppe R₂ eine (C₁- C₆)-Alkylgruppe, eine Aryl- oder Aralkylgruppe darstellt.
